(19) Europäisches Patentamt / European Patent Office / Office européen des brevets

(11) **EP 2 340 871 A2**

(12) **EUROPEAN PATENT APPLICATION**

(43) Date of publication:
06.07.2011 Bulletin 2011/27

(51) Int Cl.:
*A61N 5/10* (2006.01)

(21) Application number: **10196831.1**

(22) Date of filing: **23.12.2010**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **28.12.2009 JP 2009298045**

(71) Applicant: **Right Mfg. Co., Ltd.**
**Itabashi-ku**
**Tokyo 174-8633 (JP)**

(72) Inventor: **Nagata, Tatsuhiko**
**Tokyo 174-8633 (JP)**

(74) Representative: **Prechtel, Jörg**
**Weickmann & Weickmann**
**Patentanwälte**
**Postfach 86 08 20**
**81635 München (DE)**

(54) **Apparatus for limiting irradiation field, and linear-member driving device**

(57) An apparatus for limiting an irradiation field includes aperture leaves arranged in a width direction thereof, the aperture leaves being controlled to be unmovable in the width direction and movable in a driving direction substantially perpendicular to the width direction. The apparatus causes the aperture leaves to block radiations emitted by a source so as to provide a desired irradiation area. At least one aperture leaf is a supporting aperture leaf having a groove at a periphery thereof. The apparatus includes a linear member, one end of which is secured to the periphery of the supporting aperture leaf and the linear member engages with the groove. The apparatus also includes a restricting portion to hold the linear member between the restricting portion and the groove such that the supporting aperture leaf is controlled to be unmovable in the width direction and movable in the driving direction.

FIG. 2

EP 2 340 871 A2

## Description

This application is based on and claims the benefit of priority from Japanese Patent Application No. 2009-298045, filed on 28 December 2009, the content of which is incorporated herein by reference.

BACKGROUND OF THE INVENTION

Field of the Invention

The present invention relates to an apparatus for limiting an irradiation field that limits a field to be irradiated by radiation, and to a linear-member driving device to drive a linear member.

Related Art

Conventionally, a widely applied apparatus for limiting an irradiation field forms an irradiation field for radiation corresponding to the shape of a focal site (for example, refer to Examined Publication Number JP H7-10282).

The apparatus for limiting an irradiation field fixes a linear member, bar-shaped member or the like to an outer peripheral portion or the like of an aperture leaf that shields radiation, and drives the aperture leaf by driving these members in an axial direction.

However since the apparatus for limiting an irradiation field must drive a plurality of linear members or bar-shaped members independently in an axial direction, a space for installation of driving devices must be provided in a thickness direction of aperture leaves. As a result, the aperture leaf of the apparatus for limiting an irradiation field requires a considerable thickness, and consequently there is a limitation on the formation of a highly precise radiation irradiation range.

SUMMARY OF THE INVENTION

The present invention provides an apparatus for limiting an irradiation field that enables formation of a highly precise irradiation field for radiation, and a linear-member driving device that enables driving of a fine linear member.

The present invention solves the above problem by the following solution means. For the sake of simplicity of description, the following description will refer to reference numerals corresponding to the embodiments of the invention, however the invention is not limited thereby. The configuration described with reference to the reference numerals may be suitably modified, or at least a portion thereof may be substituted using another component.

In a first aspect of the present invention, an apparatus for limiting an irradiation field is provided. The apparatus includes a plurality of aperture leaves arranged in a width direction thereof, the aperture leaves being controlled so as to be unmovable in the width direction and movable in a driving direction substantially perpendicular to the width direction. The apparatus causes the aperture leaves to block radiations emitted by a radiation source such that the field to be irradiated is restricted to a desired area. At least one of the aperture leaves is a supporting aperture leaf having a groove at a periphery thereof. The apparatus includes a linear member, one end of which is secured to the periphery of the supporting aperture leaf and the linear member engages with the groove. The apparatus also includes a restricting portion configured to hold the linear member between the restricting portion and the groove such that the supporting aperture leaf is controlled so as to be unmovable in the width direction and movable in the driving direction.

In a second aspect of the present invention, the restricting portion of the apparatus according to the first aspect may include a roller provided with a groove on an external circumference thereof and supported rotatably in the driving direction of the supporting aperture leaf.

In a third aspect of the present invention, the apparatus according to the second aspect may further include a connection unit connecting an adjacent pair of the aperture leaves such that the adjacent pair is configured to be unmovable in the width direction and movable in the driving direction. The aperture leaves include supporting aperture leaves and at least one intermediate aperture leaf that is placed between two of the supporting aperture leaves controlled by adjacent restricting portions. The adjacent restricting portions control the intermediate aperture leaf to be unmovable in the width direction and movable in the driving direction through the two of the supporting aperture leaves and the connection unit.

In a fourth aspect of the present invention, the connection unit of the apparatus according to the third aspect may include a rolling portion disposed between the adjacent pair of the aperture leaves and a housing portion provided for at least one of the adjacent pair of the aperture leaves to accommodate the rolling portion.

In a fifth aspect of the present invention, the linear member of the apparatus according to the first aspect may include a first forming portion and a second forming portion disposed adjacent to the first forming portion. The linear member of the first forming portion may be configured in a freely supported state such that the linear member lies more apart from the periphery of the supporting aperture leaf as a position of the linear member approaches another end differing from the secured one end of the linear member. In addition, the linear member of the second forming portion may be configured in a freely supported state such that the linear member lies closer to the periphery of the supporting aperture leaf as a position of the linear member further approaches the other end from an end of the first forming portion.

In a sixth aspect of the present invention, the apparatus according to the first aspect may further include a plurality of linear member driving devices that are disposed at different positions from one another when viewed in the width direction of the aperture leaves. Each of the linear member driving devices may include stacked unit drivers each driving another end differing from one secured end of a linear member in a direction of an axial line of the linear member.

In a seventh aspect of the preset invention, each of the linear member driving devices of the apparatus according to the sixth aspect may drive a group of linear members arranged at intervals of a predetermined number of linear members arranged in the width direction of the aperture leaves.

In an eighth aspect of the present invention, each of the unit drivers of the apparatus according to the sixth aspect may include an actuator to rotationally drive a linear member. In addition, a central axis of a drive shaft of the actuator of one of the unit drivers and a central axis of a drive shaft of an actuator of at least another one of the unit drivers may be configured to be substantially coaxial.

In a ninth aspect of the present invention, the apparatus according to the eighth aspect may further include a drive force transmission unit configured to transmit a drive force of the actuator to the linear member. The drive force transmission unit may include one of a drive wire and a drive belt to which the linear member is connected and a plurality of pulleys onto which the one of the drive wire and the drive belt is wound. The actuator may be configured to rotationally drive one of the pulleys.

In a tenth aspect of the present invention, each of the unit drivers of the apparatus according to the ninth aspect may include a tension adjustment unit configured to press the one of the drive wire and the drive belt so as to adjust tension applied thereto.

In an eleventh aspect of the present invention, the apparatus according to the ninth aspect may further include a stopper, a drive force detection unit, a slippage detection unit and an output unit. The stopper is configured to restrict one of the aperture leaves from moving beyond a predetermined amount. The drive force detection unit is configured to detect a drive force transmitted by the drive force transmission unit while the one of the aperture leaves is restricted by the stopper. The slippage detection unit is configured to detect whether the one of the drive wire and the drive belt slips from the pulleys due to the transmitted drive force while the one of the aperture leaves is restricted by the stopper. The output unit is configured to output results of detection performed by the drive force detection unit and the slippage detection unit.

In a twelfth aspect of the present invention, the one of the drive wire and the drive belt of the apparatus according to the ninth aspect may be configured to slip at a surface thereof in contact with the pulleys due to the linear member receiving predetermined tension.

In thirteenth aspect of the present invention, the apparatus according to the sixth aspect may further include at least two position sensors and a controller. The position sensors differ from each other in detection of a driven position of the linear member. The controller is configured to perform at least one of reducing a driving speed, reversing a driving direction and stopping a drive for the linear member when the controller compares signals sent from the at least two position sensors and detects a predetermined amount of difference therebetween.

In a fourteenth aspect of the present invention, the at least two position sensors of the apparatus according to the thirteenth aspect may include an absolute position sensor configured to detect an absolute position of the linear member and a relative position sensor configured to detect an amount of the linear member driven with respect to a predetermined position.

In a fifteenth aspect of the present invention, a method for adjusting an apparatus for limiting an irradiation field is provided. The method includes the steps of: (a) restricting an aperture leaf by a stopper; (b) transmitting a drive force to the aperture leaf while the aperture leaf is restricted in step (a); (c) detecting the drive force transmitted to the aperture leaf in step (b) by a drive force detection unit; (d) determining whether the drive force detected in step (c) is out of a predetermined range by checking a signal sent to an output unit; (e) detecting by a slippage detection unit whether the drive force transmitted in step (b) causes one of a drive wire and a drive belt to slip from a pulley; (f) determining whether the one of the drive wire and the drive belt slips from the pulley detected in step (e) based on the signal sent to the output unit; (g) adjusting tension applied to the one of the drive wire and the drive belt. Step (g) includes the steps of: reducing the tension when in step (d), the drive force is determined equal to or greater than an upper limit of the predetermined range and in step (f), no occurrence of slippage is determined; and increasing the tension when in step (d), the drive force is determined equal to or smaller than a lower limit of the predetermined range and in step (f), an occurrence of slippage is determined.

In a sixteenth aspect of the present invention, the drive force is detected by causing the drive force detection unit to be mechanically connected with the aperture leaf , in step (c) of the method according to the fifteenth aspect.

In a seventeenth aspect of the present invention, linear member driving device for driving a plurality of linear members in directions of axial lines thereof is provided. The linear member driving device includes a plurality of stacked unit drivers, each being configured to drive each of the linear members. Each of the unit drivers includes an actuator to rotationally drive each of the linear members. A central axis of a drive shaft of the actuator and a central axis of a drive shaft of an actuator of at least another one of the unit drivers are substantially coaxially arranged.

In an eighteenth aspect of the present invention, the linear member driving device according to the seventeenth aspect may further include a drive force transmission unit configured to transmit a drive force to one of the linear members. The drive force transmission unit includes one of a drive wire and a drive belt to which the one of the linear members is connected and a plurality of pulleys onto which one of the drive wire and the drive belt is wound. The actuator drives one of the pulleys to rotate.

In a nineteenth aspect of the present invention, the linear member driving device according to the eighteenth aspect may further include a tension adjustment unit configured to press the one of the drive wire and the drive belt so as to adjust tension applied thereto.

In a twentieth aspect of the present invention, the one of the drive wire and the drive belt of the linear member driving device according to the eighteenth aspect may be configured to slip at a surface thereof in contact with the pulleys due to the one of the linear members receiving predetermined tension.

In a twenty-first aspect of the present invention, the linear member driving device according to the seventeenth aspect may further include at least two position sensors differing from each other in detection of a driven position of the one of the linear members and a controller configured to compare signals sent from the at lest two position sensors and stop driving the one of the linear members when a predetermined difference exists between the signals.

In a twenty-second aspect of the present invention, the at least two sensors of the linear member driving device according to the twenty-first aspect of the present invention, may include an absolute position sensor configured to detect an absolute position of the one of the linear,members and a relative position sensor configured to detect an amount of the one of the linear members driven with respect to a predetermined position.

According to the present invention, the following effects are obtained.

(1) Since the present invention fixes one end of a linear member to an aperture leaf, the aperture leaf can be driven by displacing the linear member in an axial direction. Furthermore, since the linear member drives a supporting aperture leaf, and displacement in a thickness direction of the supporting aperture leaf is limited, the configuration for supporting the aperture leaf can be simplified.

(2) Since the restricting portion of the present invention includes a groove on an outer periphery and is provided with a roller, it is possible to reduce the drive force for driving of the aperture leaf and implement stable driving of the aperture leaf. Furthermore since the roller rotates together with the driving of the linear member, the durability of the linear member can be improved since the linear member does not undergo sliding.

(3) Adjacent restricting portions in the present invention limit an intermediate aperture leaf through two supporting aperture leaves and a connection unit. Therefore of the plurality of aperture leaves, each restricting portion may support only one group including the supporting aperture leaves and the intermediate aperture leaf. In this manner, application of an excessive load on each restricting portion can be prevented and therefore stable driving of the aperture leaf is enabled.

(4) Since the present invention includes the rolling portion disposed between adjacent aperture leaves and the housing portion that houses the rolling portion, the intermediate aperture leaf that is disposed between supporting aperture leaves can be controlled.

(5) Since the present invention includes the first forming portion, the linear member is placed in contact with the outer peripheral portion of the aperture leaf by a biasing force, and therefore stable driving of the aperture leaf is enabled.

(6) Since the present invention includes the plurality of linear-member driving devices that is provided with the stacked unit drivers, a group including a predetermined number of linear members can be driven by one linear-member driving device independently of another group including the predetermined number of linear members. In this manner, the linear-member driving device ensures a space for installation of the unit drivers for driving of the linear members in a thickness direction of the aperture leaf. Therefore the thickness of the aperture leaf can be reduced to enable formation of a highly precise irradiation field for radiation and irradiation of small foci.

(7) Since each linear-member driving device of the present invention drives the linear members spaced at intervals corresponding to the predetermined number of linear members as a single group, a space required for the predetermined number of aperture leaves can be ensured in the thickness direction so as to install unit drivers.

(8) Since one actuator of one unit driver and another actuator of at least one other unit driver according to the present invention are configured so that central axes of drive shafts thereof are substantially concentric, two or more linear members can be driven on a single axis to enable downsizing of the apparatus.

(9) The drive force transmission unit according to the present invention is provided with the drive belt or the like and the pulley that are adapted to have a reduced thickness. In this manner, the size of the overall apparatus can be reduced.

(10) Since the present invention includes the tension adjustment unit for adjusting the tension of the drive wire or the drive belt, a suitable tension can be maintained even when stretching occurs in the drive wire or drive belt due to changes over time or the like.

(11) The present invention detects whether or not the drive wire or the drive belt and the pulley slip when the aperture leaf receives a transmitted drive force while the aperture leaf is limited by the stopper. Therefore an operator can operate the tension adjustment unit while confirming the detection result to enable improvement of an adjustment operation. The present invention prevents the application of an excessive load on the linear member by adjusting the tension of the drive belt or the like since the drive belt or the like can slip when an excessive drive force acts thereon. As a result, the provision of a disc-shaped clutch or the like is not required to prevent an excessive force on the linear member, and therefore the configuration can be simplified.

(12) The contact faces between the drive wire or drive belt and the pulley slip as a result of a predetermined tensile force applied to the linear member. Therefore damage to the apparatus can be prevented since the application of an excessive load to the linear member can be prevented.

(13) The present invention determines the driven position of the linear member based on an output of at least two detection units. When there is the predetermined difference in determined driven positions, application of at least one of reduction of the drive speed of the linear member, reversing the direction of driving, or stopping driving is executed. Therefore durability is improved, and stable driving of the aperture leaf is enabled.

(14) Since the present invention is provided with the absolute position sensor and the relative position sensor, the driven position of the aperture leaf can be determined using two different methods based on detection of an absolute position and detection of a relative position.

(15) During the tension reduction process, when it is determined that the drive force is greater than or equal to the upper limit of a required range, and the drive belt or the like and the pulley are not in a state of slippage, an adjustment is executed to reduce the tension on the belt or the like. During the tension increase process, when it is determined that the drive force is less than or equal to the lower limit of the required range, and that the drive belt or the like and the pulley are in a state of slippage, an adjustment is executed to increase the tension on the belt or the like. An operator can perform an accurate adjustment with a simple adjustment operation as described above so that the drive belt or the like and the pulley are in a state of slippage as a result of application of a drive force that is within the required range.

(16) Since a drive force is detected under mechanical connection of the drive force detection unit and the aperture leaf, the present invention allows the actual drive force to be detected, enabling accurate adjustment.

BRIEF DESCRIPTION OF THE DRAWINGS

FIG. 1 is a partial perspective view of an apparatus for limiting an irradiation field according to a first embodiment;
FIG. 2 is a view of the apparatus for limiting an irradiation field according to the first embodiment as seen from a thickness direction T of an aperture leaf;
FIG. 3 is a sectional view (a sectional view along line III-III of FIG. 2) seen from a driving direction of the aperture leaf according to the first embodiment;
FIG. 4 illustrates a state in which a linear member is guided according to the first embodiment;
FIG. 5A shows the linear member according to the first embodiment that is freely mounted on the aperture leaf, and FIG. 5B shows the linear member delivered in a direction of closing the aperture leaf;
FIG. 6 is an enlargement of the portion VI of FIG. 3;
FIG. 7 is an exploded perspective view of a linear-member driving device according to the first embodiment;
FIG. 8 is a sectional view of the linear-member driving device according to the first embodiment (a sectional view along line VIII-VIII of FIG. 7);
FIG. 9 is a view of a unit driver according to the first embodiment seen from the thickness direction T of the aperture leaf, and shows a linear sensor;
FIG. 10A illustrates a setup applied to a method 1 for adjusting a slip drive force in the apparatus for limiting an irradiation field according to the first embodiment, and FIG. 10B shows a graph that describes the relationship between a drive voltage V1 and a measured drive force F1;
FIG. 11 is a flowchart describing the method 1 for adjusting a slip drive force according to the first embodiment;
FIG. 12A illustrates a setup applied to a method 2 for adjusting a slip drive force in the apparatus for limiting an irradiation field according to the first embodiment, and FIG. 12B shows a graph that describes the relationship between a drive voltage V2 and a calculated drive force F2;
FIG. 13 is a flowchart describing the method 2 for adjusting a slip drive force according to the first embodiment;
FIG. 14 is a sectional view seen from a direction of driving of an aperture leaf unit according to a second embodiment (corresponding to FIG. 3 according to the first embodiment);
FIG. 15 is an enlarged view of the dotted line portion of FIG. 14;
FIG. 16 is a view of an apparatus for limiting an irradiation field according to a third embodiment seen from a thickness direction T of an aperture leaf (corresponding to FIG. 2 according to the first embodiment);

FIG. 17 is a sectional view seen from a driving direction of the aperture leaf according to the third embodiment (a sectional view along line XVII - XVII of FIG. 16), and illustrates a perspective view of a roller;

FIG. 18 is a sectional view of the proximity of a support member 306 according to the third embodiment seen from the direction of driving, and is an enlarged view of the portion XVIII in FIG. 17; and

FIG. 19 is a sectional view of the proximity of a support member 305 according to the third embodiment seen from the direction of driving, and is an enlarged view of the portion XIX in FIG. 17.

DETAILED DESCRIPTION OF THE INVENTION

Embodiments of the present invention will be described below in further detail by making reference to the figures.

Embodiment 1

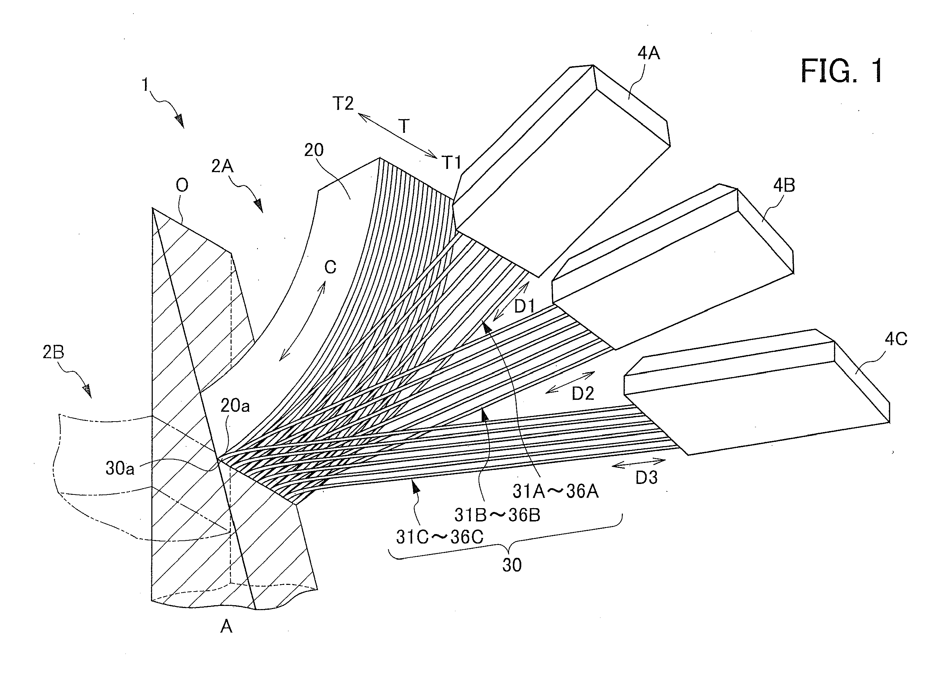

FIG. 1 is a partial perspective view of an apparatus for limiting an irradiation field 1 according to a first embodiment.

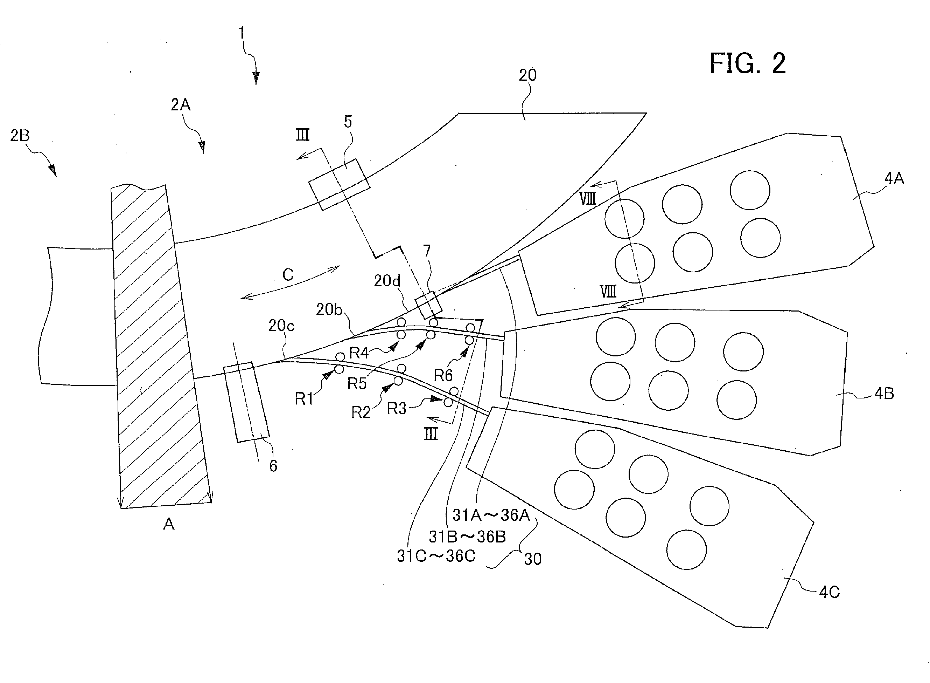

FIG. 2 is a view of the apparatus for limiting an irradiation field 1 according to the first embodiment seen from a thickness direction T (T1) of an aperture leaf 20.

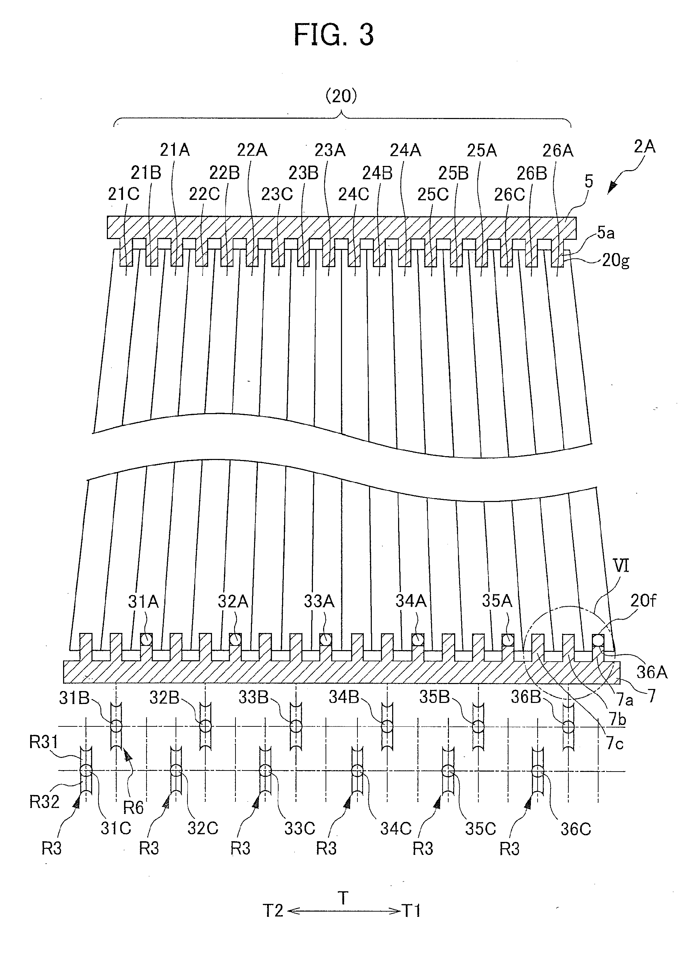

FIG. 3 is a sectional view (a sectional view along line III-III of FIG. 2) of an aperture leaf unit 2A according to the first embodiment seen from a driving direction.

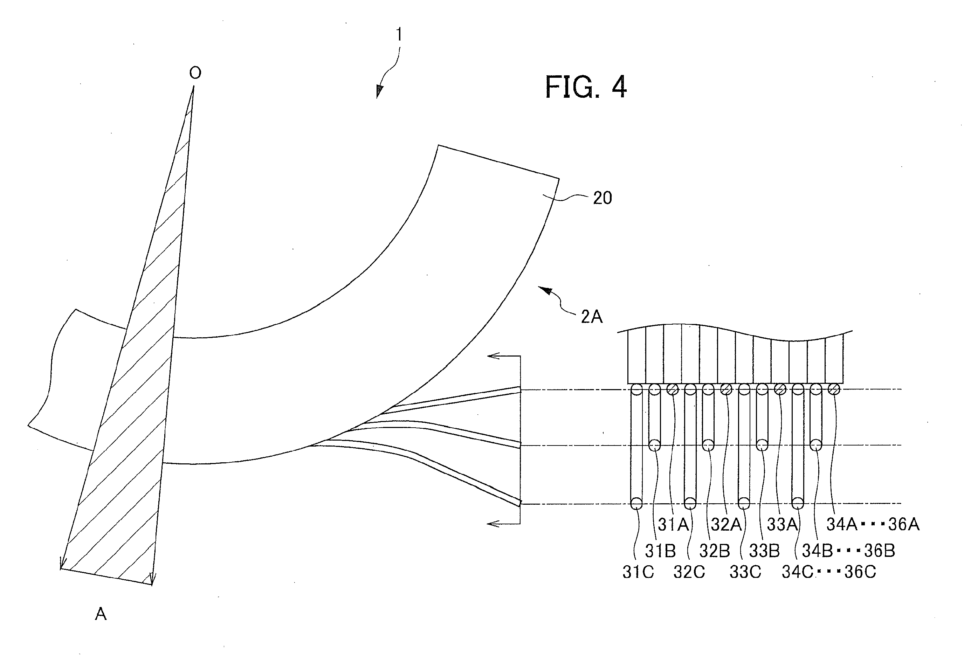

FIG. 4 illustrates a state in which a linear member 30 is guided according to the first embodiment.

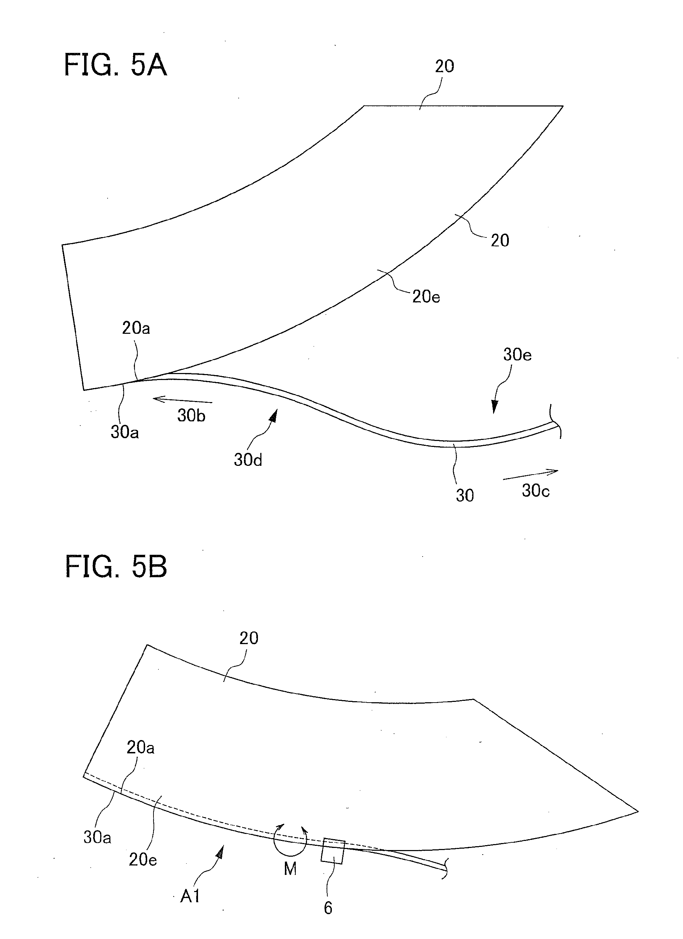

FIG. 5A shows the linear member 30 according to the first embodiment that is freely mounted on the aperture leaf 20. FIG. 5B shows the linear member 30 delivered in a direction of closing the aperture leaf 20.

As shown in FIG. 1 and FIG. 2, the apparatus for limiting an irradiation field 1 shields radiation irradiated from a radiation source O such that the irradiation field is limited to a desired range. The apparatus for limiting an irradiation field 1 includes oppositely disposed aperture leaf units 2A and 2B for shielding radiation, linear members 30 (31A - 36A, 31B - 36B, 31C - 36C), three linear-member driving devices 4A - 4C that drive the linear members 30, guide rollers R1 - R6, and support members 5 - 7.

In the following description, the configuration of the aperture leaf unit 2A will be described, which is applicable to a similar configuration of the aperture leaf unit 2B.

As shown in FIG. 3, the aperture leaf unit 2A includes eighteen aperture leaves 20 (21A - 26A, 21B - 26B, 21C - 26C) that are arrayed with respect to a thickness direction T of the unit 2A. The sectional shape of an aperture leaf 20 is substantially in the form of a parallelogram. The thickness direction T will be described as a direction that is orthogonal to a driving direction C.

The aperture leaf 20 is a thin plate-shaped member, and as shown in FIG. 2, has a shape of a sector when seen from the thickness direction T. The aperture leaf 20 is supported rotatably about the radiation source O by support members 5 - 7, as will be described later. The direction of rotation is the driving direction C of the aperture leaf 20. A material used for the aperture leaf 20 is tungsten or the like to shield radiation emitted from the radiation source O.

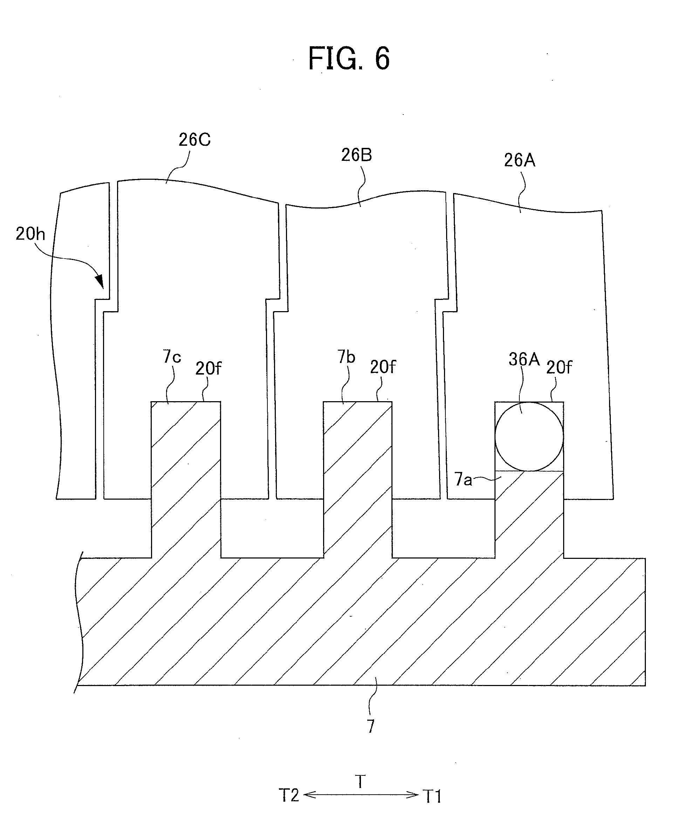

The aperture leaves 20 are disposed in the order of aperture leaf 21C, 21B, 21A, 22C, 22B, 22A, 23C, 23B, 23A, 24C, 24B, 24A, 25C, 25B, 25A, 26C, 26B, 26A from a front side (thickness direction T2) to an inner side (thickness direction T1) in FIG. 1. Adjacent aperture leaves 20 are disposed to form a minute space, and a step portion 20h (FIG. 6) is formed so that the radiation does not pass through the space.

As shown in FIG. 1, the linear members 30 are composed of eighteen wires that drive the aperture leaves 20. The linear members 30 are disposed in the order of linear member 31C, 31B, 31A, 32C, 32B, 32A, 33C, 33B, 33A, 34C, 34B, 34A, 35C, 35B, 35A, 36C, 36B, 36A from the front side to the inner side in FIG. 1. The linear members 30 are disposed on a one-to-one basis so as to drive the aperture leaves 20 in the order of disposition. In other words, for example, the linear member 31A drives the aperture leaf 21A, the linear member 31B drives the aperture leaf 21B, and the linear member 31C drives the aperture leaf 21C. One end 30a of each linear member 30 is fixed in a tangential direction to a fixing portion 20a on an outer periphery of an aperture leaf 20. The linear members 30 are driven in their axial directions (directions D1 to D3 as shown by arrows).

The other end of each linear member 30 (the other end different from the end described above) is connected to any one of linear-member driving devices 4A - 4C, and is driven in the axial direction. Since each linear member 30 drives an aperture leaf 20 while undergoing resilient deformation, it is composed of a resilient member (for example, a wire of a continuous metal line, a wire rope of twisted wires, a hollow pipe, or the like).

As shown in FIG. 2, the linear-member driving devices 4A - 4C are disposed in different positions as viewed from the thickness direction T1. More particularly, the linear-member driving devices 4A - 4C are disposed substantially radially so that the direction of letting out the linear members 30 is oriented substantially in a direction of the fixing portion 20a. Of the linear-member driving devices 4A - 4C, the linear-member driving device 4A drives the linear members 31A - 36A, the linear-member driving device 4B drives the linear members 31B - 36B, and the linear-member driving device 4C drives the linear members 31C - 36C.

As shown in FIG. 1 and FIG. 4, the linear-member driving devices 4A - 4C alternately drive the linear members 30, so that each of the linear-member driving devices 4A - 4C drives the linear members 30 at intervals of two linear members 30. That is to say, of the linear members 30 that are arrayed as a group in the thickness direction T, the linear-member driving devices 4A - 4C drive the linear members 31A - 36A, 31B - 36B and 31C - 36C that are arrayed spaced at intervals of two members.

In this manner, the linear-member driving devices 4A - 4C maintain a space for two aperture leaves 20 in the thickness direction T in order to dispose devices to drive the linear members 30 (unit drivers 51 - 56 or the like, as will be described later). Since the apparatus for limiting a radiation irradiation field 1 can reduce a pitch between adjacent linear members 30, it is possible to drive an aperture leaf 20 with a lower thickness. Accordingly, it is possible to form the contour of an irradiation field A to be more definite.

Inner construction of the linear-member driving devices 4A - 4C will be described later.

As shown in FIG. 2, guide rollers R1 - R3 guide the linear members 31C - 36C to separate earlier from the outer peripheries of aperture leaves 20 rather than the linear members 31A - 36A and 31B - 36B. In a similar manner, guide rollers R4 - R6 guide the linear members 31B - 36B to separate earlier from the outer peripheries of other aperture leaves 20 than the linear members 31A - 36A. As shown in FIG. 3, for example, the guide roller R3 includes rollers R31 and R32 that are provided with a V-shaped groove on an outer periphery, respectively and supported rotatably about an axis in the thickness direction T. The rollers R31 and R32 are disposed in opposite positions so that a pair of rollers R31 and R32 sandwiches each of the linear members 31C - 36C. The other guide rollers are similar to the guide roller R3 described above.

In this manner, the guide rollers R1 - R6 guide the linear members 31B - 36B and 31C - 36C, and subject the linear members 31B - 36B and 31C - 36C to resilient deformation such that the linear members 31B - 36B and 31C - 36C are movably supported in their axial directions. Furthermore when the linear members 31B - 36B and 31C - 36C are subjected to loads in their axial directions during a driving operation, the guide rollers R1 - R6 prevent buckling of the linear members 31B - 36B and 31C - 36C, which may occur between the linear member driving devices 4B, 4C and positions 20b, 20c where the linear members 31B - 36B and 31C - 36C start separating from the aperture leaves 20.

In this connection, the linear members 31A - 36A are not provided with guide rollers. Since the distance between a position 20d where the linear members 31A- 36A start separating from the aperture leaves 20 and the linear member driving device 4A is small, the linear members 31A - 36A do not require guiding or prevention of buckling by the guide rollers.

However, when design conditions or the like require an increase in the distance, guide rollers may be provided for the linear members 31A - 36A for the purpose of guiding and preventing buckling of the linear members 31A - 36A.

As shown in FIG. 5A, a linear member 30 includes, in a free state not guided by a guide roller, a first forming portion 30d and a second forming portion 30e. The linear member 30 of the first forming portion 30d is formed to lie more apart from an outer peripheral portion 20e (edge portion) as a position of the linear member 30 approaches from one end 30a (direction of an arrow 30b) fixed to the aperture leaf 20 to the other end (direction of an arrow 30c). The linear member 30 of the second forming portion 30e is formed to lie closer to the outer peripheral portion 20e as a position of the linear member 30 approaches further from an end portion of the first forming portion 30d towards the other end. The first forming portion 30d is substantially arc-shaped to come into contact with the outer peripheral portion 20e, and the second forming portion 30e is substantially arc-shaped and bent to an opposite side to the first forming portion 30d.

When the linear members 30 are guided by the guide rollers R1 - R6 along with the effect provided by the first forming portion 30d, a biasing force causes the linear members 30 to abut with outer peripheral portions 20e of the aperture leaves 20. Accordingly, it is possible to prevent the linear members 30 from rising from the outer peripheral portions 20e, thereby enabling stable driving of the aperture leaves 20.

The inventors of the present invention have confirmed by a test demonstrating that such rising is more effectively prevented by providing the second forming portion 30e in addition to the first forming portion 30d.

Next, the supporting structure of the aperture leaves 20 will be described with reference to FIG. 2, FIG. 3 and FIG. 6.

FIG. 6 is a sectional view seen from the driving direction C of the proximity of a support member 7 according to the first embodiment, and is an enlargement of the portion IV of FIG. 3.

As shown in FIG. 2, the aperture leaves 20 are supported by a support member 5 provided on an inner peripheral side and two support members 6 and 7 provided on an outer peripheral side.

As shown FIG. 3, each aperture leaf 20 has grooves 20f and 20g formed along a peripheral direction on an outer peripheral portion (edge) and an inner peripheral portion. The groove 20f is a recess bored into the outer peripheral portion toward an inner radial side, and the groove 20g is a recess bored into the inner peripheral portion toward an outer radial side. The grooves 20f and 20g have bottom faces parallel with the outer peripheral portion (edge) and the inner peripheral portion, respectively and have inner side surfaces that are orthogonal to the bottom faces. They are machined using a blade such as an end mill, for example.

A linear member 30 is engaged with a groove 20f of an aperture leaf 20 from the fixing portion 20a (refer to FIG. 1) to position 20b, 20c or 20d where the linear member 30 separates from the aperture leaf 20 (refer to FIG. 2).

The support member 5 includes protruding portions 5a formed along the peripheral direction to be engaged with grooves 20g. The support member 5 restricts the aperture leaves 20 from displacing to an inner radial side and in the thickness direction T. In addition, the support member 5 allows the aperture leaves 20 to displace in the driving direction C.

The support member 6 has protruding portions that are oriented in a direction different from the protruding portions 5a of the support member 5 and engaged with grooves 20f. The support member 6 prevents the aperture leaves 20 from displacing in an outer radial direction and thickness direction T of the aperture leaves 20. In addition, the support member 6 allows the aperture leaves 20 to displace in the driving direction C.

Similarly as shown in FIG. 6, the support member 7 includes protruding portions 7b and 7c that are oriented in a different direction from the protruding portions 5a, and furthermore includes restricting portions 7a that have a smaller degree of protrusion than the protruding portions 7b and 7c. A restricting portion 7a sandwiches the linear member 36A with a groove 20f so as to prevent the aperture leaf 26A from displacing towards an outer radial side and in the thickness direction T (a direction orthogonal to the driving direction) of the aperture leaf 26A. In addition, the restricting portion 7a allows the aperture leaf 26A to displace in the driving direction C.

The support member 7 includes six sets, each including the restricting portion 7a and the protruding portion 7b and the protruding portion 7c as a single set, in the thickness direction T. Six restricting portions 7a and the linear members 31A-36A support the aperture leaves 21A - 26A (supporting aperture leaves) and execute guiding of the aperture leaves 21A - 26A in the driving direction C.

In this manner, the linear members 31A - 36A drive the aperture leaves 21A - 26A and prevent the aperture leaves 21A - 26A from displacing toward the outer radial side and in the thickness direction T of the aperture leaves 21A - 26B in cooperation with the restricting portions 7a. Accordingly, the setup for driving and retaining the aperture leaves 21A - 26B can be simplified.

Next the architecture of the linear-member driving device 4A will be described. The linear-member driving devices 4B and 4C have a similar architecture as the linear-member driving device 4A and therefore description thereof will not be repeated.

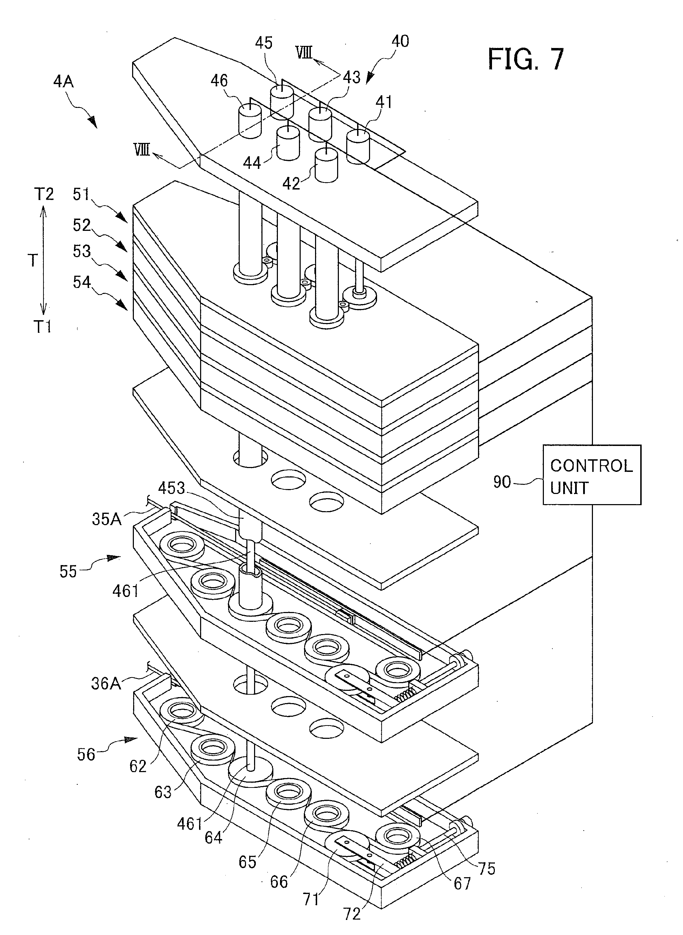

FIG. 7 is an exploded perspective view of the linear-member driving device 4A according to the first embodiment.

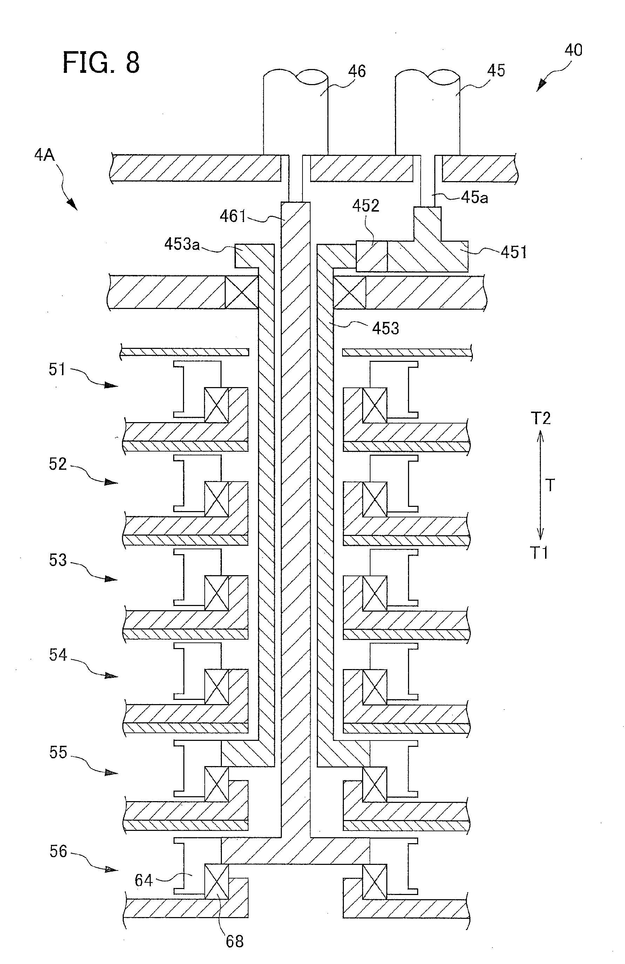

FIG. 8 is a sectional view of the linear-member drive device 4A according to the first embodiment (a sectional view along line VIII-VIII of FIG. 7).

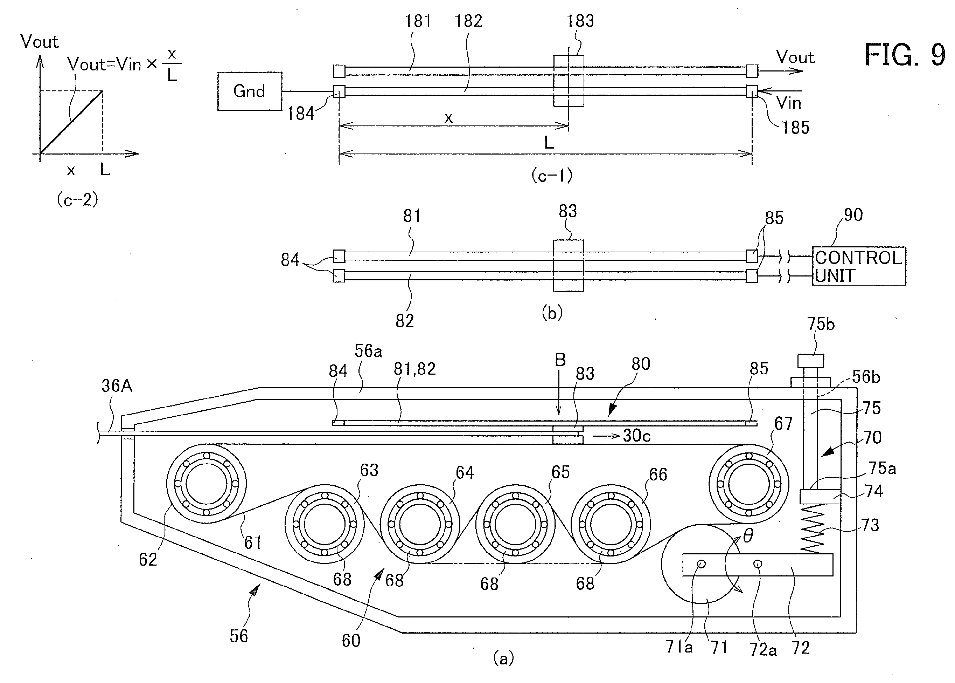

In FIG. 9, (a) shows a unit driver 56 according to the first embodiment seen from the thickness direction T1, and (b) shows a linear sensor 80 (seen from the direction of an arrow B shown in (a)).

As shown in FIG. 7, the linear-member driving device 4A includes an actuator 40 and six unit drivers 51 - 56.

The actuator 40 includes six motors 41 - 46, and drive shafts or the like connected to the motors.

A motor 46 for example is a step motor. As shown in FIG. 8, a rotation shaft is directly connected to a drive shaft 461, and a rotational force is transmitted to the drive shaft 461. A rotation shaft 45a of a motor 45 is connected through a gear 451 and an idle gear 452 to a drive shaft 453 provided with a gear portion 453a, such that the rotation force is transmitted to the drive shaft 453.

The drive shaft 453 is formed as a hollow cylinder to enclose the drive shaft 461, and is disposed concentric to the drive shaft 461. As a result, the linear-member driving device 4A transmits the drive force of the two motors 45 and 46 about a single axis independently to the two unit drivers 55 and 56, and enables independent driving of the two linear members 35A and 36A, as shown in FIG. 7.

Similarly, the drive forces of the motors 41 and 42 are transmitted independently to the two unit drivers 51 and 52, and the drive forces of the motors 43 and 44 are transmitted independently to the two unit drivers 53 and 54.

Since the linear-member driving device 4A drives the six unit drivers 51 - 56 independently about three axes, it is possible to reduce the size in a plane vertical to the thickness direction T.

Next, the unit drivers 51 - 56 will be described.

Each unit driver 51 - 56 is configured to drive each respective linear member 31A - 36A. In addition, as shown in FIG. 7, each unit driver 51-56 is a box-shaped unit that has a low thickness with respect to the thickness direction T. The linear-member driving device 4A includes six unit drivers 51 - 56 stacked with respect to the thickness direction T.

As described above, the linear-member driving device 4A ensures spare space for two aperture leaves 20 in the thickness direction T to dispose each unit driver 51 - 56. The inventors of the present invention prepared an actual apparatus for limiting an irradiation field for radiation, and confirmed that the thickness of each unit driver 51 - 56 could be reduced to the level of substantially 5 mm and the thickness of the aperture leaf 20 could be reduced to the level of substantially 1.6 mm.

As shown in FIG. 9, the unit driver 56 includes a case 56a, a drive force transmission unit 60, a tension adjustment unit 70, and a linear sensor 80. Since other unit drivers 51 - 55 have a similar construction, description thereof will be omitted.

The drive force transmission unit 60 includes a drive belt 61, and six pulleys 62 - 67 having the drive belt 61 coiled thereon.

The drive belt 61 for example is a flat belt such as a stainless steel belt. An end portion on the other end of the linear member 36A (in the direction of an arrow 30c) is fixed by soldering or the like to the drive belt 61. Alternatively, the linear member 36A may be fixed to the drive belt 61 so that the end portion of the linear member 36A protrudes to the right of the figure. In other words, it may be sufficient if a portion of the other end of the linear member 36A is fixed to the drive belt 61.

Each pulley 62 - 67, which is formed in a circular ring, is supported rotatably about an axis in the thickness direction T with respect to the case 56a through a circular roller bearing 68. Of the six pulleys 62 - 67, a pulley 64 is rotatably driven by the drive shaft 461 and the motor 46 of the actuator 40 (refer to FIG. 8), and rotates the drive belt 61. The outer peripheral surface of the drive pulley 64 (the contact surface with the drive belt 61) is provided with a fine uneven texture to transmit the drive force efficiently and prevent slip of the drive belt 61 during driving.

When the linear member 31A is subjected to a predetermined tensile force, the drive belt 61 slips on the contact surface of the pulley 64 since the drive belt 61 is a flat belt. In this manner, the linear-member driving device 4A prevents damage to the apparatus when an excess load is applied to the linear member 36A.

The tension adjustment unit 70 includes a tension adjustment pulley 71, a support arm 72, a spring 73, a spring support 74, and a screw 75.

The tension adjustment pulley 71 is retained by a support arm 72 to be rotatable about a rotation shaft 71a.

The support arm 72 is supported on the case 56a to be rotatable about a shaft 72a (refer to an arrow θ).

The spring 73 is a compression coil spring disposed between the support arm 72 and the spring support 74. The spring 73 is not limited to a compression coil spring. Alternatively, a plate spring, a helical torsion spring, or the like may be used according to an installation space or the like.

The spring support 74 is supported on the case 56a to be movable in a vertical direction in the figure.

The screw 75 is a male threaded portion that is threadably engaged with a female threaded portion 56b provided on the case 56a, and passes through the case 56a. The screw 75 adjusts a position of the spring support 74 by abutment of a screw tip 75a with the spring support 74.

With the configuration described above, the tension adjustment unit 70 transmits the force of the extended spring 73 to the supporting arm 72 such that a rotational force in a clockwise direction in the figure is applied to the supporting arm 72. Accordingly, the tension adjustment unit 70 causes the tension adjustment pulley 71 to press the drive belt 61, thereby adjusting the tension of the drive belt 61.

Since the linear-member driving device 4A includes the tension adjustment unit 70 that adjusts the tension of the drive belt 61, a suitable tension can be maintained even when the drive belt 61 is stretched as a result of changes over time.

When it is necessary to change coiling of the drive belt 61 such that a drive belt 61 of a different length is adopted, it is possible to absorb differences in length in the drive belt 61.

Since a head 75b of the screw 75 is exposed on an outer portion of the case 56a, tension adjustment can be easily performed from an external portion of the case 56a.

Next, a control method and method of detecting a driven position of the linear member 36A, that is to say, the aperture leaf 26A, will be described.

As shown in FIG. 9, the unit driver 56 has a linear sensor 80 connected to a control unit 90.

The linear sensor 80 is an absolute position sensor provided on the unit driver 56. The linear sensor 80 includes two electrically resistive wires 81 and 82 and a short bar 83. The electrically resistive wires 81 and 82 are disposed in parallel to a direction of driving the linear member 36A, and have an electric resistance value that increases proportional to a length thereof. The short bar 83 is fixed to the linear member 36A, and slides on the electrically resistive wires 81 and 82 together with the driving of the linear member 36A.

The electrically resistive wires 81 and 82 are electrically connected to the control unit 90. When the short bar 83 moves as a result of tracking the displacement of the linear member 36A, the control unit 90 detects a variation in the electric resistance value. Accordingly, the control unit 90 determines the position of the short bar 83, in other words, the driven position of the linear member 36A and the aperture leaf 26A. Contact points 84 and 85 are respectively provided on both ends of the linear sensor 80. The electric resistance value increases as the short bar 83 approaches the contact points 84, that is to say, as the linear member 36A is let out. On the other hand, the electric resistance value decreases as the short bar 83 approaches the contact points 85, in other words, the linear member 36A is retracted.

A short circuit is caused when the short bar 83 comes into contact with the contact points 84 and 85, and the detected electric resistance value becomes zero. When the electric resistance value has become zero, the control unit 90 determines that the linear member 36A is positioned at a limit of the driving range, and stops the rotation of the motor 46 (refer to FIG. 8) .

As shown in (c-1) of FIG. 9, a copper wire 181 and an electrically resistive wire 182 may be adopted, and a contact point 184 on a tip of the electrically resistive wire 182 may be connected to the ground Gnd (potential 0). In this case, the electric resistance value of the copper wire 181 is 0.

Suppose that the electrically resistive wire 182 is assigned to a side of an input voltage $V_{in}$, the copper wire 181 is assigned to a side of an output voltage $V_{out}$, the electrically resistive wire 182 has an overall length L, and a length from the contact point 184 of the electrically resistive wire 182 to the short bar 83 is x, the following relationship holds (Refer to (c-2) of FIG. 9):

$$V_{out} = V_{in} \cdot (x/L)$$

When a constant voltage is applied to the side of the input voltage $V_{in}$, and the output voltage $V_{out}$ is measured, the control unit 90 can simply obtain a length x, that is to say, a position of the short bar 83.

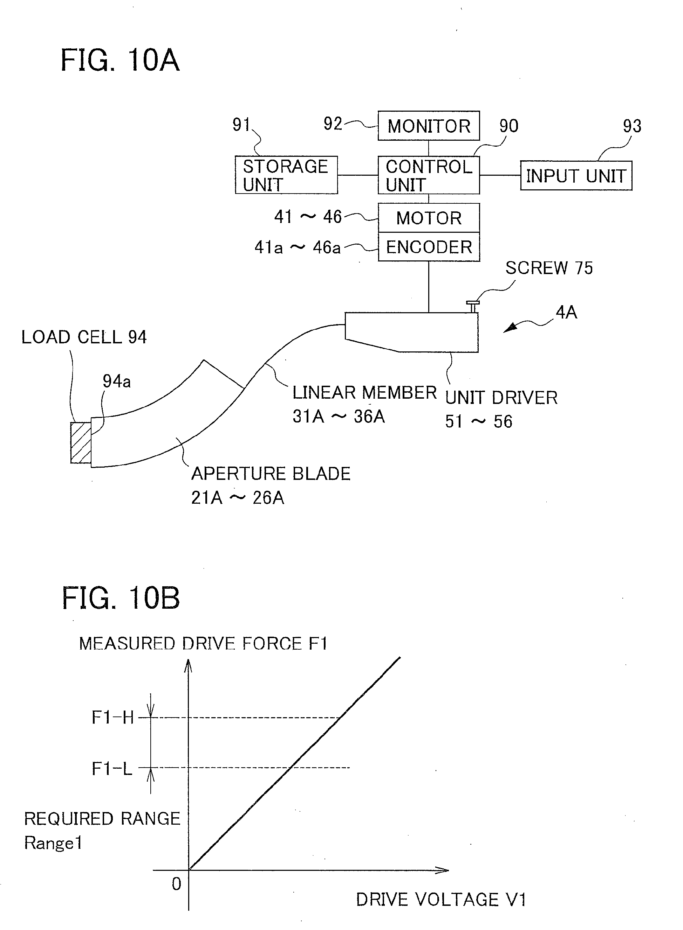

An encoder 46a (refer to FIG. 10) is provided on the motor 46 (refer to FIG. 8) in order to detect a rotation angle of the drive shaft 461. The encoder 46a is a relative position sensor that detects an amount of the linear member 36A driven relative to a predetermined position with higher resolution than the linear sensor 80. The encoder 46a includes for example an LED (light-emitting diode), a photosensitive element, and a code wheel that rotates integrally with the drive shaft 461. The encoder 46a converts light and dark associated with light rays, which are emitted by the LED, reflected by the code wheel and detected by the photosensitive element, to a pulse signal and outputs it to the control unit 90.

An example of resolution executed by the encoder 46a is as follows: the encoder can detect an amount of 1/256 mm (that is to say, 3.9 $\mu$m) of the driven linear member 36A for a case where a pulse number of 256 is outputted during one rotation of the drive shaft 461 and the corresponding displacement of the linear member is 1 mm.

For example, when the aperture leaf 26A is driven by a predetermined drive amount, the control unit 90 determines a predetermined position of the linear member 36A from the output of the linear sensor 80, and causes the motor 46 to be driven for a count corresponding to the pulses of the drive amount. For the exemplary case described above where the drive amount is set to 1 mm, the control unit 90 may cause the motor 46 to be driven until the encoder 46a outputs 256 pulses.

Alternatively, the predetermined position may be determined based on the output of a limit switch provided at a predetermined interval to detect the driven position of the linear member 36A without using the linear sensor 80.

In this manner, the linear-member drive device 4A executes highly precise driving of the aperture leaf 26A using the linear sensor 80 and the encoder 46a, and thereby enables highly accurate formation the radiation irradiation field A.

When the driven position of the linear member 36A is determined based on the pulse signal of the encoder 46a, the control unit 90 also determines the driven position of the linear member 36A based on the output of the linear sensor 80. In other words, the control unit 90 determines the driven position of the linear member 36A in two manners based on the output of the detection units of two different measurement methods, relative position detection and absolute position detection of the driven position of the linear member 36A. When there is a predetermined difference in the two determined driven positions, the control unit 90 causes the motor 46 to stop, stopping the driving of the linear member 36A.

As an example of a difference in the determination of the two driven positions, a situation may be assumed in which a foreign object is sandwiched between opposite aperture leaves 20, and the drive belt 61 and the pulley 64 slip from each other to cause the motor 46 to undergo idle running. Under the circumstance described above, the problem arises that components of the apparatus are subjected to loads to cause damage, or that the radiation from the radiation source O cannot be controlled to desired intensity.

In contrast, the linear-member driving device 4A that stops driving of the linear member 36A when there is a predetermined difference in the two determined driving positions enables not only improvement of the durability of the apparatus, but also stable driving of the aperture leaf 26A.

In this connection, a drive unit that is composed of a motor 41, a gear, or the like normally undergoes a rattling such as a backlash, or the like. Accordingly, when there is a change in the direction of rotation of the motor 46, for example, an error may occur between the driven position of the linear member 36A determined by the control unit 90 based on the pulse signal of the encoder 46a, and the actual driven position.

The linear-member driving device 4A according to the first embodiment prevents a reduction in the drive accuracy caused by backlash by executing control as described hereafter.

When the control unit 90 causes the motor 46 to rotate in a predetermined direction (hereinafter referred to as "positive rotation"), and subsequently executes rotation in the opposite direction (hereinafter referred to as "opposite rotation"), the control unit 90 continues to count the pulse number of the encoder 46a until a change in the output of the linear sensor 80 occurs. In addition, the control unit 90 applies a positive rotation after applying an opposite rotation to the motor 46, and counts the pulse number of the encoder 46a. The control unit 90 executes a plurality of these measurements in order to improve the measurement accuracy, stores the results in a memory (not shown), and halves the average value of the measurements such that the pulse number is determined according to an amount of backlash.

When the rotation direction of the motor 46 is changed, for example, the control unit 90 determines a position of the driven linear member 36A by incorporating a pulse number corresponding to the backlash amount and improves the drive accuracy of the linear member 36A. When the pulse number corresponding to the backlash is two pulses, for example, the control unit 90 causes the motor 46 to rotate until two more pulses than normal have been counted to drive the linear member 36A.

The backlash amount may differ depending on the position of the driven linear member 36A. As a result, the control unit 90 may measure the pulse number corresponding to the backlash amount at a plurality of positions such as both ends or the central portion and the like of the driving range of the linear member 36A. Accordingly, the control unit 90 may execute separate use of the pulse number corresponding to the backlash in response to the driven position of the linear member 36A.

As described above, the apparatus for limiting a radiation irradiation field 1 according to the first embodiment enables a reduction in the thickness of the aperture leaf 20 by displacement of the linear member 30 in an axial direction, and therefore enables highly accurate formation of the irradiation field of the radiation.

Since the linear members 31A - 36A not only drive the aperture leaves 21A - 26A, but also limit the displacement of the aperture leaves 21A - 26A, the configuration for retaining the aperture leaves 21A - 26A can be simplified.

Furthermore, the linear-member driving devices 4A - 4C drive the linear members 30 with a space corresponding to two linear members 30, respectively. Accordingly, the linear-member driving devices 4A - 4C drive the fine linear members 30 while maintaining a spare space for two aperture leaves 20 in the thickness direction T.

The linear-member driving devices 4A - 4C also drive two linear members 30 on a single axis, and thereby enable downsizing of the apparatus.

Method of Adjusting Slip Drive Force

Next, the method of adjusting the slip drive force of the apparatus for limiting a radiation irradiation field 1 according to the first embodiment will be described.

The "slip drive force" indicates a drive force at which the drive belt 61 and the pulley 62 - 67 begin to slip from each other when the drive force applied to an aperture leaf 20 is gradually increased while the aperture leaf 20 is restricted from displacement.

The apparatus for limiting a radiation irradiation field 1 employs adjustment of the slip drive force with two different methods, methods 1 and 2 for adjusting slip drive force as described below.

Method 1 for Adjusting Slip Drive Force

FIG. 10A illustrates the configuration applied to a method 1 for adjusting a slip drive force in the apparatus for limiting an irradiation field 1 according to the first embodiment. FIG. 10B shows a graph that describes the relationship between a drive voltage V1 and a measured drive force F1.

As shown in FIG. 10A, the apparatus for limiting an irradiation field 1 includes encoders 41a - 46a, a storage unit 91, a monitor 92 (output portion), an input unit 93, and a load cell 94 (drive force detection unit).

The linear-member driving devices 4B and 4C have a similar configuration as the linear-member driving device 4A, and therefore description thereof will not be repeated.

Each of the encoders 41a - 46a is a sensor that detects a rotation angle of each of the drive shafts of the motors 41 - 46 (refer to FIG. 7).

The storage unit 91 includes semiconductor memory elements, hard disks and the like for storing information and programs required for operation of the apparatus for limiting an irradiation field 1. The control unit 90 cooperates with the hardware described above to realize functions according to the present invention by reading and executing various types of programs stored in the storage unit 91. The monitor 92 is a display apparatus such as a liquid-crystal display.

The monitor 92 displays the output of the encoders 41a - 46a, and the load cell 94, drive voltages V1 of the motors 41 - 46, and the like.

The input unit 93 includes a keyboard, or the like.

The load cell 94 includes a piezoelectric element and measures a drive force in a closing direction of the aperture leaves 20. The load cell 94 measures the pressure applied to a measurement face 94a by an aperture leaf 20, that is to say, the drive force of the aperture leaf 20. The load cell 94 is disposed to abut with the face in a closing direction of the aperture leaf 20, and functions as a limiting unit that imposes a limit so that the aperture leaf 20 does not displace in a closing direction. The drive force measured by the load cell 94 hereafter is denoted as a measured drive force F1.

As shown in FIG. 10B, when the motors 41 - 46 are DC motors, the measured drive force F1 linearly increases if the drive voltage V1 is increased.

The required range Range1 as shown in FIG. 10B is a predetermined range for the drive force that takes into account such as the design strength for preventing buckling of the linear members 31A - 36A and the drive force required for driving the linear members 31A - 36A. The required range Range1 is defined by an upper limit F1 - H and a lower limit F1 - L.

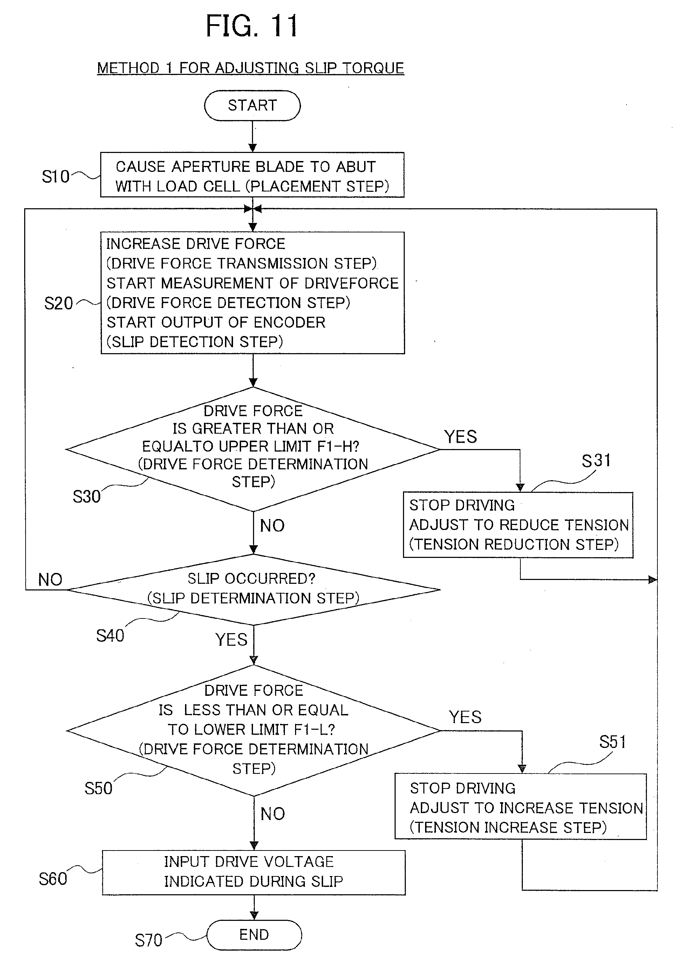

FIG. 11 is a flowchart describing the method 1 for adjusting a slip drive force according to the first embodiment.

In step S10 (hereinafter simply referred to as "S"), an operator operates the input unit 93 to drive the aperture leaf 21A and place the tip of the aperture leaf 21A in contact with the load cell 94 (placement step). In this manner, the aperture leaf 21A and the load cell 94 are mechanically connected with each other.

In S20, the operator operates the input unit 93 to increase the drive voltage so as to increase the drive force (drive force transmission step).

The control unit 90 starts measuring the drive force with the load cell 94 (drive force detection step).

The encoder 41a detects the rotation state of the motor 41 (slip detection step). The rotation state of the motor 41 is outputted to the monitor 92.

In S30, while checking the output of the load cell 94 with the monitor 92, the operator determines whether the measured drive force F1 is greater than or equal to an upper limit F1-H (drive force determination step). If the measured drive force F1 is greater than or equal to the upper limit F1-H (S30:YES), the processing proceeds to S31. If the measured drive force F1 is less than the upper limit F1-H (S30:NO), the processing proceeds to S40.

In S31, the operator operates the input unit 93 to stop driving operations.

The operator operates the screw 75 (refer to FIG. 9) so that the tension of the drive belt 61 (refer to FIG. 9) is reduced. In other words, friction between the drive belt 61 and the pulleys 62 - 67 is reduced by reducing the tension of the drive belt 61, and thereby the slip drive force is reduced (tension reduction step). Thereafter, the operation from S10 is repeated.

In S40, while checking the output of the encoder 41a with the monitor 92, the operator determines whether the drive belt 61 and the pulleys 62 - 67 have slid (slipped) from each other (slip determination step). When slippage occurs, the motor 41 rotates although the output of the encoder 41a does not increase. In this manner, the operator can determine whether slippage has occurred by confirming the output of the encoder 41a.

If the drive belt 61 and the pulleys 62 - 67 undergo slippage (S40: YES), the processing proceeds to step S50, and on the other hand, if the drive belt 61 and the pulleys 62 - 67 have not slipped (S40: NO), the processing from S20 is repeated.

In S50, the operator determines whether the measured drive force F1 is less than or equal to the lower limit F1-L based on the output of the load cell 94 on the monitor 92. (drive force determination step).

If the measured drive force F1 is less than or equal to the lower limit F1-L (S50:YES), the processing proceeds to step S51, and if the measured drive force F1 is greater than the lower limit F1-L (S50:NO), the processing proceeds to step S60.

In S51, the operator operates the input unit 93 to stop the driving operation. The operator operates the screw 75 (refer to FIG. 9) to increase the tension of the drive belt 61 (refer to FIG. 9) (tension increase step). In other words, the friction between the drive belt 61 and the pulleys 62 - 67 is increased by increasing the tension of the drive belt 61 to increase the slip drive force. Thereafter the operation from S10 is repeated.

When slippage occurs between the upper limit F1-H and the lower limit F1-L, the processing proceeds to S60. The operator operates the input unit 93 to input the drive voltage. The control unit 90 causes the inputted drive voltage to be stored in the storage unit 91. Then the processing proceeds to S70 to complete the adjustment process.

The operator performs similar adjustment on the aperture leaves 22A - 26A.

The apparatus for limiting a radiation irradiation field 1 may apply an excessive load to the linear member 30 if an excessive drive voltage is applied in case a foreign object has intruded in the sliding portion to prevent slippage. As a result, the intrusion of a foreign object may be a cause of damage for the apparatus.

However since the apparatus for limiting a radiation irradiation field 1 allows the drive belt 61 and the pulleys 62 - 67 to slip from each other with the adjustment method as described above, it is possible to prevent damage to the apparatus, thereby increasing the safety of operation.

Furthermore when the apparatus for limiting a radiation irradiation field 1 is in operation, the control unit 90 causes the motors 41 - 46 corresponding to the aperture leaves 21A - 26A to stop if an excess drive voltage is applied that is greater by a predetermined amount (for example at least 20% greater) than the drive voltage stored in S60. In this manner, for example, even if the drive belt 61 and the pulleys 62 - 67 do not slip from each other, the apparatus for limiting a radiation irradiation field 1 can prevent itself from suffering damage, and can improve stable operation. Method 2 for Adjusting Slip Drive Force

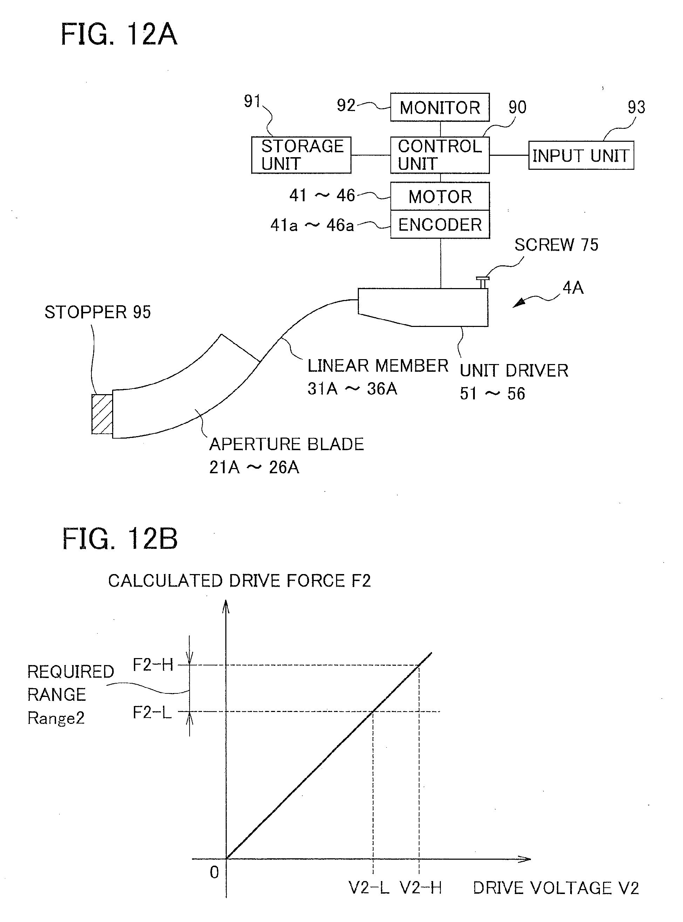

FIG. 12A describes the configuration applied to a method 2 for adjusting a slip drive force in the apparatus for limiting an irradiation field 1 according to the first embodiment. FIG. 12B shows a graph that describes the relationship between a drive voltage V2 and a calculated drive force value F2.

In the description and the figures below, those portions that execute the processes and functions that are the same as the method 1 described above are denoted by the same reference numerals, or numerals having the same reference numerals attached at an end thereof. Overlapping description will be omitted as appropriate.

As shown in FIG. 12A, the apparatus for limiting an irradiation field 1 includes a stopper 95 in substitution for the load cell 94.

As described above, when DC motors are used for the motors 41 - 46, the drive force undergoes a linear increase when the drive voltage is increased.

As shown in FIG. 12B, a drive force can be calculated based on the drive voltage V2.

The required range Range2 of the drive force is predetermined similar to the required range Range1. The upper limit V2-H and the lower limit V2-L of the drive voltage V2 corresponding to the upper limit F2-H and the lower limit F2-L of the required range Range2 may be calculated in advance.

Accordingly, drive forces acting on the aperture leaves 21 - 26 can be indirectly detected by acquiring calculated drive forces F2 based on drive voltages V2 of the motors 41 - 46, if a load cell 94 is not introduced.

In addition, it is possible to determine whether the drive force reaches the upper limit F2-H or the lower limit F2-L of the required range Range2 by confirming whether the drive voltage V2 reaches the upper limit V2-H or the lower limit V2-L.

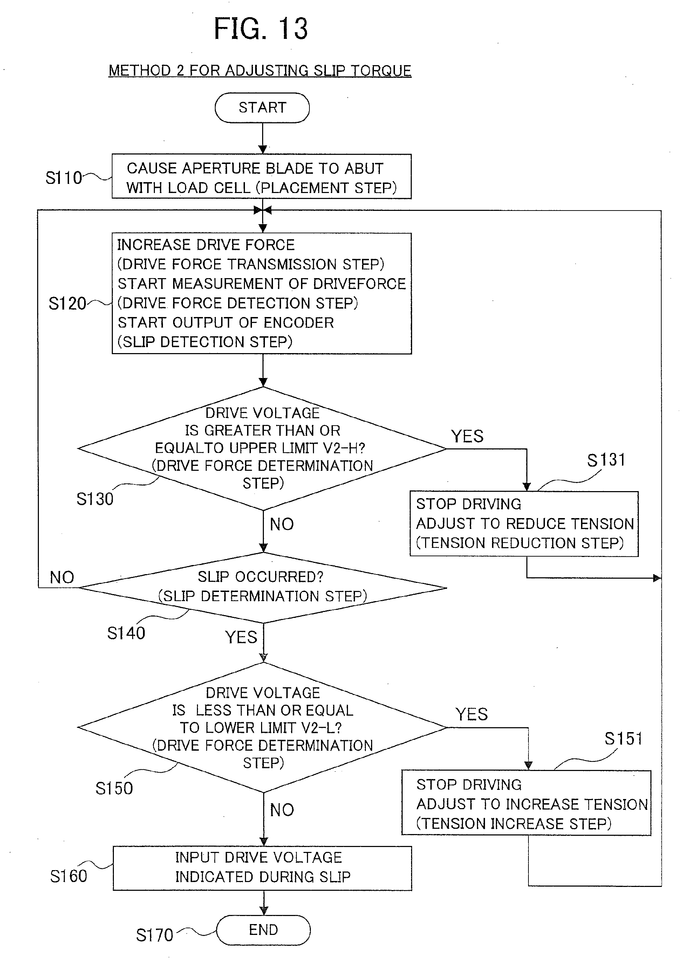

FIG. 13 is a flowchart describing a method 2 for adjusting a slip drive force according to the first embodiment.

In S120, the control unit 90 indirectly detects a drive force according to a drive voltage V2 applied to the motor 41. For example, an operator may confirm the drive voltage V2 by reading a value given by a voltmeter displayed on the monitor 92.

In S130, the operator determines whether the drive force has reached the upper limit by checking whether the drive voltage is equal to or greater than the upper limit V2-H.

In S150, the operator determines whether the drive force has reached the lower limit by checking whether the drive voltage is less than or equal to the lower limit V2-L.

Since the method 1 and the method 2 for adjusting a slip drive force impart effects as described below, the operator may select either method according to the purpose of the adjustment and the constraints of adjustment time.

Since the method 1 for adjusting a slip drive force measures the actual drive forces of the aperture leaves 21 - 26 that are free of deviations in the motors 41 - 46, it is possible to perform precise adjustment.

Since the method 2 for adjusting a slip drive force enables simplification of the configuration of the apparatus, and an operation to install the load cell 94 is not required, the adjustment operation is simple.

Second Embodiment

A second embodiment of an apparatus for limiting a radiation irradiation field to which the present invention is applied will be described below.

In the description and the figures below, those portions that execute the processes and functions that are the same as the method 1 for adjusting a slip drive force described above are denoted by the same reference numerals, or numerals having the same reference numerals attached to an end thereof, and overlapping description will be omitted as appropriate.

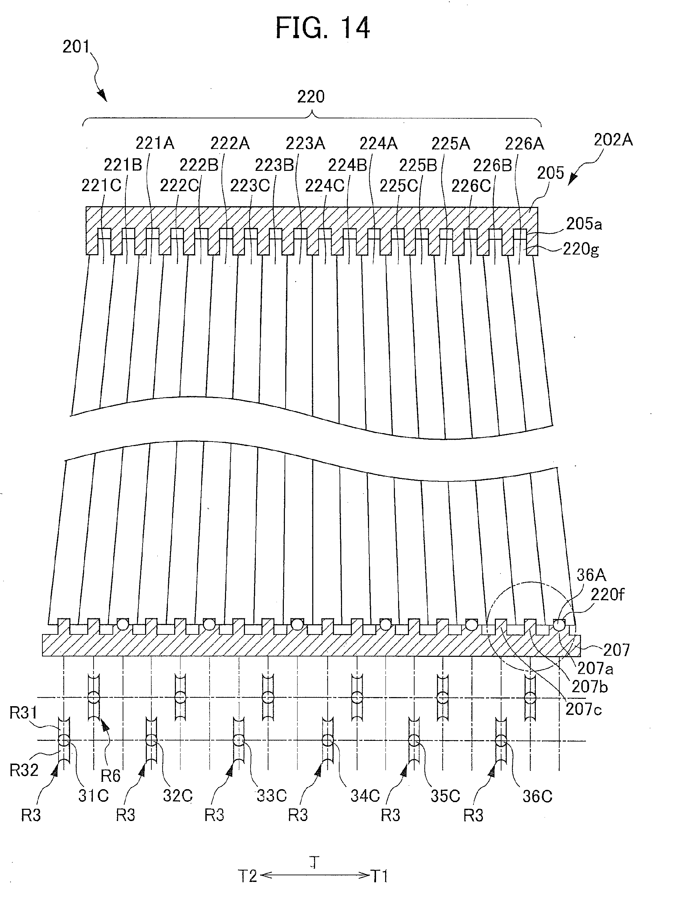

FIG. 14 is a sectional view seen from a direction of driving of an aperture leaf unit 202A of an apparatus for limiting a radiation irradiation field 201 according to a second embodiment (corresponding to FIG. 3 according to the first embodiment).

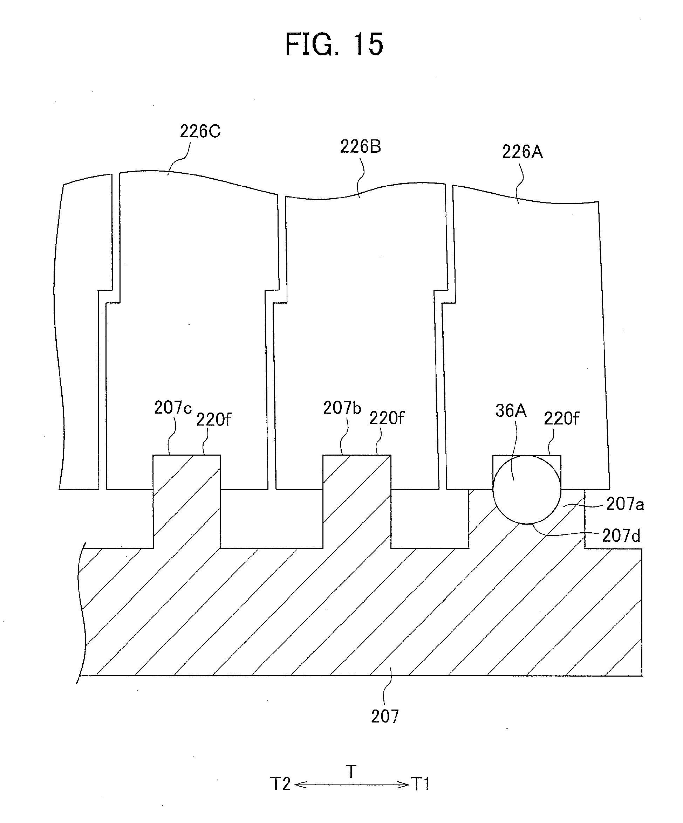

FIG. 15 is an enlarged view of the dotted line portion in FIG. 14.

As shown in FIG. 14, a protruding portion 220g is formed on an aperture leaf 220 along a peripheral direction on an inner peripheral portion in order to limit displacement with respect to a radially inner direction and a thickness direction T. A support portion 205 has grooves 205a that are formed along the peripheral direction such that the grooves 205a engage with protruding portions 220g. The aperture leaf 220 is manufactured by machining or the like of a material such as tungsten or the like. Since the protruding portion 220g protrudes from the aperture leaf 220, processing is easier than the groove 20g (refer to FIG. 3).

A groove 220f is formed on an outer peripheral portion (edge) of the aperture leaf 220 in a peripheral direction in order to limit displacement in a radially outer direction and the thickness direction T.

As shown in FIG. 15, the groove 220f is formed to be more shallow than the groove 20f (refer to FIG. 6) according to the first embodiment, so that processing with an end mill or the like is better performed.

A support member 207 includes restricting portions 207a - 207c.

A restricting portion 207a includes a groove 207d that engages with a linear member 36A and prevents the linear member 36A from displacing with respect to the radially outer direction and the thickness direction T. The linear member 36A engages with the groove 220f of an aperture leaf 226A, and prevents the aperture leaf 226A from displacing with respect to the radially outer direction and the thickness direction T.

Restricting portions 207b and 207c are adapted to directly engage with grooves 220f of the aperture leaves 226B and 226C similar to the first embodiment.

In other words, the support member 207 supports aperture leaves 220 as follows: Restricting portions 207a support and indirectly restrict the six aperture leaves 221A - 226A through the linear members 31A-36A. The restricting portions 207b and 207c directly support the six aperture leaves 221B - 226B and six aperture leaves 221C - 226C, respectively.

In this manner, the apparatus for limiting a radiation irradiation field 201 according to the second embodiment enables restriction of the aperture leaf 220 with the shallower groove 220f. Accordingly, it is possible to implement better processing of the aperture leaf 220.

Embodiment 3

A third embodiment of an apparatus for limiting a radiation irradiation field to which the present invention is applied will be described below.

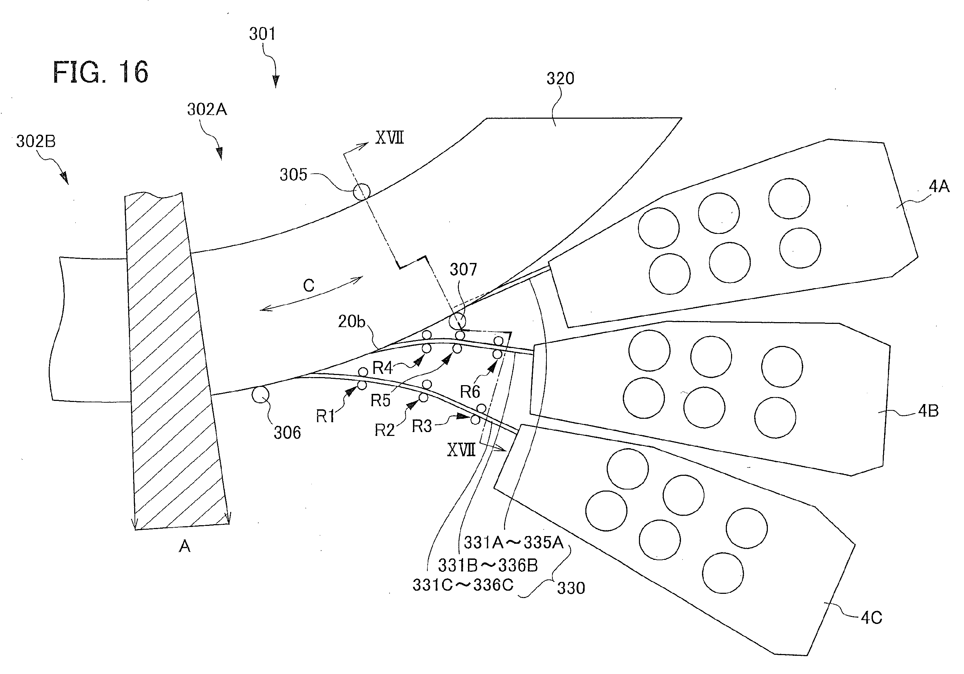

FIG. 16 is a view of an apparatus for limiting an irradiation field 301 according to a third embodiment seen from a thickness direction T of aperture leaves 320 (corresponding to FIG. 2 according to the first embodiment).

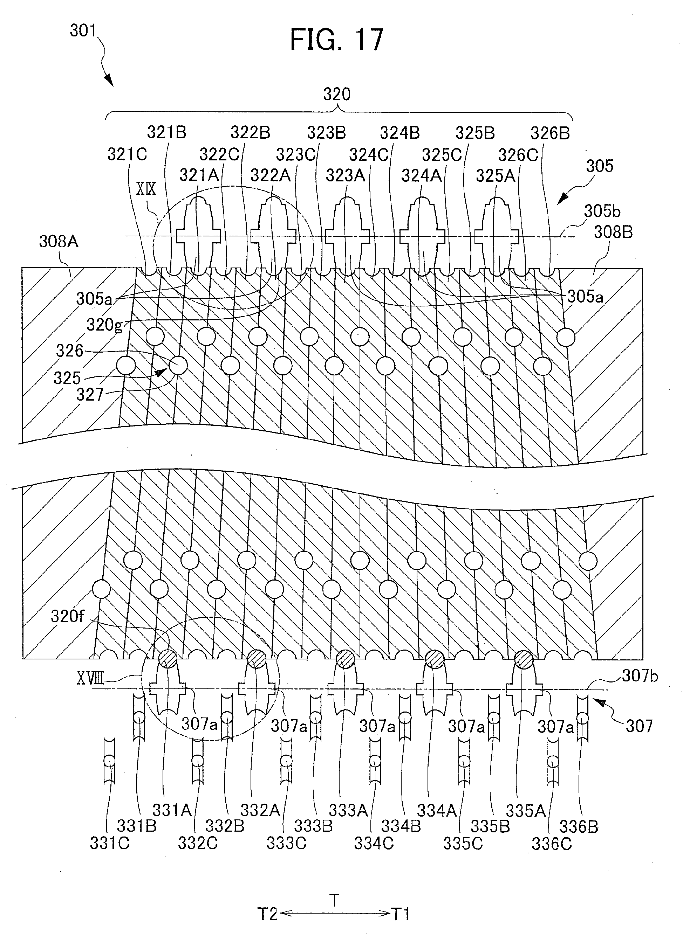

FIG. 17 is a sectional view seen from a driving direction C of an aperture leaf unit 302A according to the third embodiment (a sectional view along line XVII - XVII as shown in FIG. 16), and illustrates a perspective view of rollers 307a.

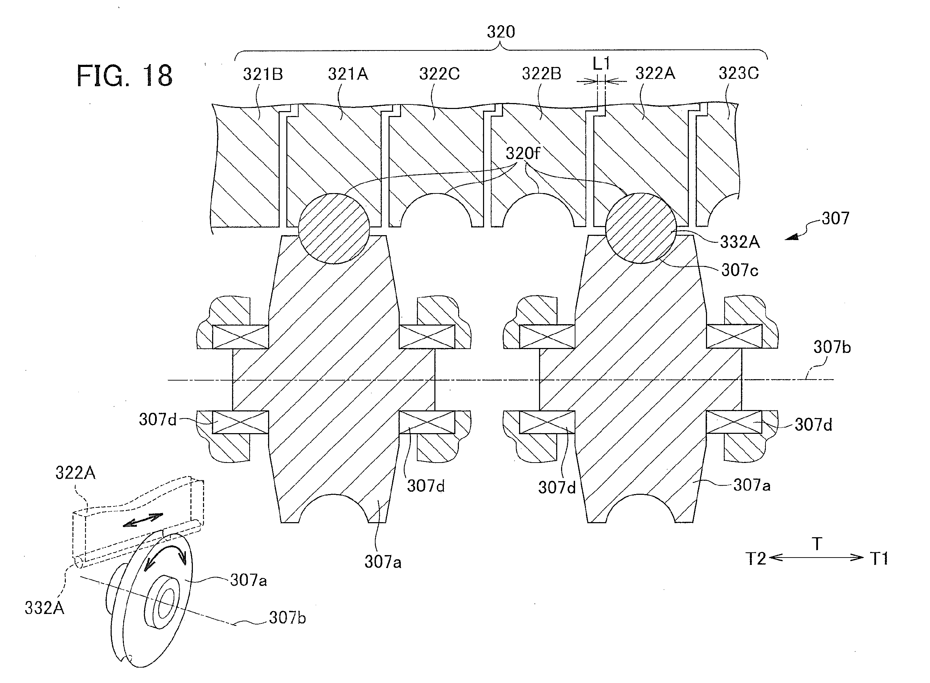

FIG. 18 is a sectional view of the proximity of a support member 306 according to the third embodiment seen from the driving direction C, and is an enlarged view of the portion XVIII in FIG. 17.

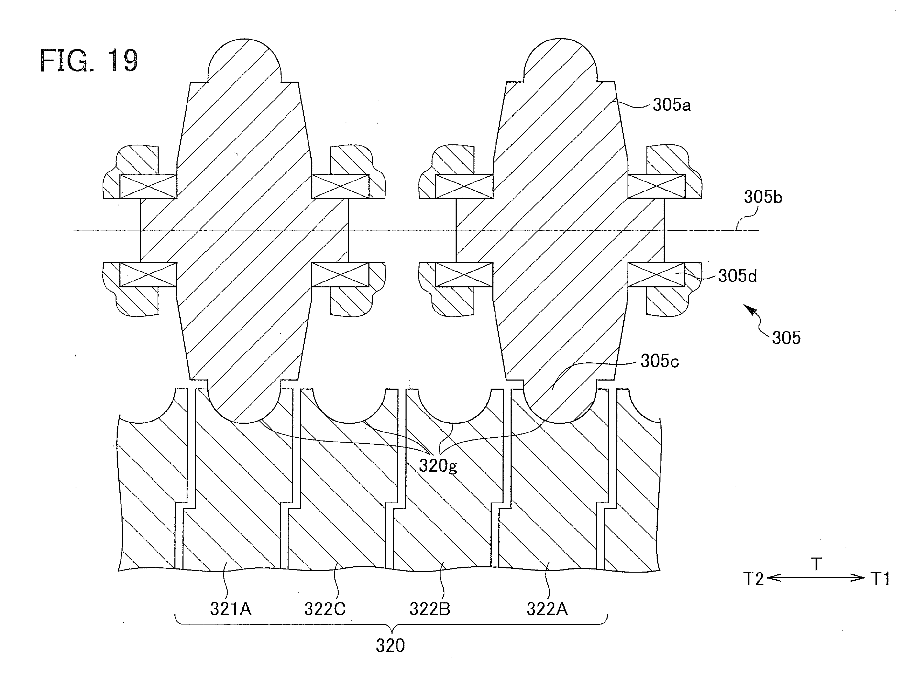

FIG. 19 is a sectional view of the proximity of a support member 305 according to the third embodiment seen from the driving direction C, and is an enlarged view of the portion XIX in FIG. 17.

As shown in FIG. 16 and FIG. 17, the apparatus for limiting an irradiation field 301 includes aperture leaves and linear members by one respectively less than the apparatus for limiting an irradiation field 1 of the first embodiment. It includes seventeen aperture leaves 320 (321A - 321C, 322A - 322C, 323A - 323C, 324A - 324C, 325A - 325C, 326B, 326C), and seventeen linear members 330 (331A - 331C, 332A - 332C, 333A - 333C, 334A - 334C, 335A - 335C, 336B, 336C).

In the apparatus for limiting an irradiation field 301, the linear member driving device 4A (refer to FIG. 1) drives five linear members 331A - 335A. In contrast, linear-member driving devices 4B and 4C (refer to FIG. 1) drive six linear members 331B - 336B and 331C - 336C, respectively similar to the first embodiment.

The apparatus for limiting an irradiation field 301 includes connection units 325, a support member 305, a support member 306, a support member 307, and end support portions 308A and 308B.

As shown in FIG. 17, each aperture leaf 320 has grooves 320f and 320g along peripheral directions on an outer peripheral portion (edge) and an inner peripheral portion. The grooves 320f and 320g are each shaped like a recess formed on the outer peripheral portion and the inner peripheral portion of each aperture leaf 320, the section of the recess being semicircular. The sectional shape is not however limited thereby, and for example, as in the first embodiment, the sectional shape may be formed to have a bottom face parallel to the outer peripheral portion and the inner peripheral portion.

Each connection unit 325 is a portion for movably supporting and connecting adjacent aperture leaves 320. Each connection unit 325 includes a ball 326 (roller portion) and a ball housing portion 327.

The ball 326 is disposed between adjacent aperture leaves 320.

The ball housing portion 327 is a groove that accommodates the ball 326. The ball housing portion 327 provides a groove in which the respective side faces of the thickness direction T of adjacent aperture leaves 320 are indented, and therefore has a sectional shape like a circular hollow. Although not shown in the figures, when viewed from the thickness direction T, the ball housing portion 327 is shaped as an arc having a length that corresponds to the drive stroke of an aperture leaf 320. A plurality of balls 326 are housed arranged in an arcuate shape in a single ball housing portion 327. The circular diameter of the sectional shape of the ball housing portion 327 is substantially the same as the diameter of the ball 326. Adjacent aperture leaves 320 sandwich to accommodate the balls 326 and engage with the balls 326, respectively. The sectional shape of the ball housing portion 327 is not limited to a circular shape. As long as the balls 326 are accommodated and engaged with the ball housing portion 327, the section may for example be a V-shaped groove.

The ball housing portion 327 is engaged with the balls 326, so that adjacent aperture leaves 320 are disposed with a fine space L1 (refer to FIG. 18). In addition, the adjacent leaves 320 are mutually limited from displacing towards a radially outer side and in the thickness direction T, and controlled to be movable in the driving direction C.

As shown in FIG. 17 and FIG. 18, the support member 307 includes five rollers 307a (restricting portions) and five pairs of bearings 307d disposed in the thickness direction T. The five rollers 307a and five pairs of bearings 307d have the same configuration, and therefore the following description will typically describe one roller 307a and one pair of bearings 307d that support an aperture leaf 322A (supporting aperture leaf) as shown in FIG. 18.

The roller 307a has a columnar shape, and includes a central shaft 307b oriented in the thickness direction T. The roller 307a is rotatably supported by the one pair of bearings 307d about the central shaft 307b. In other words, the roller 307a is supported rotatably in the driving direction C of the aperture leaf 322A.

A groove 307c is provided on the entire circumference of the roller 307a (refer to the perspective view shown in FIG. 18).

The sectional shape of the groove 307c as seen from the driving direction C is semicircular, and the radius of the semicircular shape is substantially equal to the radius of the linear member 322A to engage with each other. The groove 307c of the roller 307a and the groove 320f of the aperture leaf 322A as described above sandwich the linear member 322A. In this manner, the roller 307a limits the linear member 332A from displacing towards the radially outer side and in the thickness direction T. The linear member 332A limits the aperture leaf 320 from displacing towards the radially outer side and in the thickness direction T.

In other words, the roller 307a of the support member 307 indirectly limits the aperture leaf 322A through the linear member 332A.

The linear member 332A does not slip with respect to the roller 307a that is rotatable in the driving direction C, since the roller 307a rotates together with the driving of the linear member 332A. In this manner, the roller 307a enables the force required for driving to be less than the restricting portion 207a in the second embodiment, and increases the durability of the linear member 332A.

As will be described later, the one pair of bearings 307d is fixed to the housing of the apparatus for limiting an irradiation field 301 such that the one pair of bearings 307d is movable together with the aperture leaf 320 and the like when the direction of emitted radiation is changed.

The reason that the support member 307 is provided with only five rollers 307a is to reduce the thickness of the aperture leaf 320 to enable a finer contour of the irradiation field for radiation. In other words, since the space in the thickness direction T is limited to implement the finer contour of the irradiation field for radiation, it is not possible to ensure a space for installation of rollers 307a that directly support all the aperture leaves 320.

The configuration of the support member 306 as shown in FIG. 16 is similar to the configuration of the support member 307, and detailed description will be omitted.

As shown in FIG. 19, the support member 305 includes rollers 305a and bearings 305d.

Each roller 305a includes a protruding portion 305c.

The protruding portion 305c has a sectional shape of a semicircle as viewed from the driving direction C, and is configured to directly engage with the groove 320g. Other aspects of each roller 305a are the same as the roller 307a, and each roller 305a is rotatably supported by a bearing 305d.

The roller 305a, the protruding portion 305c of which is engaged with the groove 320g, prevents displacement of the aperture leaf 320 in the radially inner direction and the thickness direction T and controls the aperture leaf 320 to be movable in the driving direction C.

With the configuration described above, the support members 305 - 307 prevent the five aperture leaves 321A - 326A from displacing with respect to the radial direction and the thickness direction T, and control them to be movable in the driving direction C.

Next, the support of aperture leaves 320 disposed between a pair of aperture leaves (supporting aperture leaves, e.g. 321A and 322A) limited by rollers 307a will be described.

As shown in FIG. 18, the aperture leaves 322A and 321A are disposed to sandwich aperture leaves 322B, 322C (intermediate aperture leaves). Since balls 326 (refer to FIG. 17) are installed between aperture leaves 322A, 322C, 322B and 321A, the aperture leaves 322A and 321A supported by the rollers 307a prevent the aperture leaves 322B and 322C from displacing towards the radially outer side and in the thickness direction T, and control them to be movable in driving direction C.

As described above, adjacent rollers 307a indirectly support the aperture leaves 322B and 322C through the aperture leaves 322A and 321A and the balls 326.

As shown in FIG. 17, the end support portions 308A and 308B are disposed on respective outer sides of the aperture leaves 321C and 326B. The end support portions 308A and 308B press to support the aperture leaves 321C and 326B externally in the thickness direction T.

The end support portion 308A supports the aperture leaf 321C from the thickness direction T1 through balls 326. The aperture leaf 321A is supported by the support members 305 - 307.

As a result, the end support portion 308A and the support members 305 - 307 prevent the aperture leaves 321B and 321C disposed between the end support portion 308A and the aperture leaf 321A, and control them to be movable in the driving direction C.

Similarly, the end support portion 308B and the support members 305 - 307 that support the aperture leaf 325A limit the aperture leaves 326B and 326C.

Next, a variation to the irradiation direction of radiation of the apparatus for limiting an irradiation field 301 will be described.

The apparatus for limiting an irradiation field 301 is adapted to change the irradiation direction for radiation by a driving mechanism (not shown). The feature of the apparatus described above is provided to allow irradiation of radiation from various directions with respect to an affected portion of a subject lying on his or her back. The driving mechanism causes seventeen aperture leaves 320, seventeen linear members 330, the end support portions 308A and 308B, the linear member driving devices 4A - 4C, the support members 305 - 307, and the like to displace and rotate together with each other in the apparatus for limiting an irradiation field 301. The thickness direction T of the aperture leaf 320 may incline from a horizontal direction, or be oriented in a vertical direction.

When the direction of emitted radiation is varied in this manner, the apparatus for limiting an irradiation field 301 allows loads applied to the seventeen aperture leaves 320 to be supported without concentration by using the rollers 305a and 307a, and the like as described above.

Suppose in FIG. 18, for example, the irradiation direction of radiation is changed, so that the aperture leaves 320 are disposed such that the thickness direction T is vertical, the thickness direction T1 downward and the thickness direction T2 upward. The roller 307a that supports the leaf 322A is only subjected to a load from the three aperture leaves 322A, 322B and 322C, and is not subjected to a load from the aperture leaf 321A and other aperture leaves 321B and the like that are disposed above (in the thickness direction T2)the aperture leaf 321A.

In this manner, the apparatus for limiting an irradiation field 301 reduces the load applied to the roller 307a and variation in the drive force for driving the aperture leaves 320 exerted by the linear member driving devices 4A - 4C. Accordingly the apparatus 301 enables stable driving of the aperture leaves 320.

It may be an alternative that a single roller 307a is provided for each aperture leaf 320. However, as described above, when the contour of the irradiation field must be formed with high precision, it is not possible to dispose a single roller 307a for a single aperture leaf 320 since the space in the thickness direction T is limited.

In other words, the apparatus for limiting an irradiation field 301 implements a highly precise contour of the irradiation field by the following techniques: The thickness of an aperture leaf 320 is reduced. The rollers 305a and 307a, and the like are disposed spaced with intervals of two aperture leaves 320 in order to enable stable driving of the aperture leaves 320. Accordingly, the rollers 305a and 307a are configured to support the aperture leaves 322B and 322C disposed between two aperture leaves 322A and 321A through the aperture leaves 322A and 321A and the balls 326.

In the present embodiment, although an example has been described in which two aperture leaves 320 are disposed between two aperture leaves 322A, 321A, the invention is not thereby limited. For example, the number may be suitably set according to the level of accuracy of the irradiation field, the thickness of the aperture leaf 320, or the installation space for the rollers 305a and 307a.

Modified Embodiment

Although the embodiments of the present invention have been described above, the present invention is not limited by the embodiments as described above. Various modifications or variations as in the modified embodiment as described below may be performed within the technical scope of the present invention. The effect stated in the embodiment is merely exemplary of an optimal effect produced by the present invention, and the effect of the present invention is not limited to that described in the embodiment. Although the embodiment described above and the variation described below may be suitably employed in combination, detailed description thereof is omitted.

(1) Although in the embodiments, an example of the shape of the aperture leaf is shown as fan-shaped, the present invention is not limited thereby. For example, the shape of the aperture leaf may be rectangular.

(2) In the embodiments, although an example is shown in which the unit driver uses the drive belt (flat belt) to drive the linear member, the present invention is not limited thereby. For example, in substitution for the drive belt, a wire-shaped member (drive wire) may be used, or a synchronized-belt, chain, or the like may be used.

(3) In the embodiments, although an example is shown in which the linear-member driving device drives two linear members by two shafts about a single axis, the present invention is not limited thereby. For example, three or more linear members may be driven by three or more shafts overlapped about a single axis, thereby further downsizing the apparatus.

(4) In the embodiments, although an example is shown in which the encoder is provided in the motor, and the control unit determines the driven position of the linear member based on the output of the encoder, the present invention is not limited thereby. For example, the control unit may determine the driven position of the linear member based on a pulse number inputted to a stepping motor.

(5) In the embodiments, although an example is shown in which the slip drive force is controlled by the drive voltage, the present invention is not limited thereby. For example, a drive current value may be used to control the slip drive force.

(6) In the first embodiment, although an example is shown in which the method of adjusting the slip drive force limits the displacement in a closing direction of the aperture leaf, the present invention is not limited thereby. As required, displacement in an opening direction of the aperture leaf may be limited.

(7) In the embodiments, although an example is shown in which the groove of the aperture leaf includes the bottom surface that is parallel to the outer peripheral portion and the inner peripheral portion and the inner side face that is orthogonal to the bottom surface, or an example in which the sectional surface is semicircular, the present invention is not limited thereby. For example, the sectional shape of the groove of the aperture leaf may include a bottom portion that is parallel to the outer peripheral portion and the inner peripheral portion, and a side that expands into a trapezoid shape as it extends towards the opening. Alternatively, the sectional shape may be formed as a V-shaped groove.

(8) In the embodiments, although an example is shown in which the structure for supporting the aperture leaf is such that the aperture leaf has the groove and the support member (or the roller) includes a protruding portion that engages with the groove, the present invention is not limited thereby. For example, the structure for supporting the aperture leaf may be opposite to the example described above such that the aperture leaf includes a protruding portion and the supporting member (or the roller) has a groove that engages with the protruding portion.

An apparatus for limiting an irradiation field includes aperture leaves arranged in a width direction thereof, the aperture leaves being controlled to be unmovable in the width direction and movable in a driving direction substantially perpendicular to the width direction. The apparatus causes the aperture leaves to block radiations emitted by a source so as to provide a desired irradiation area. At least one aperture leaf is a supporting aperture leaf having a groove at a periphery thereof. The apparatus includes a linear member, one end of which is secured to the periphery of the supporting aperture leaf and the linear member engages with the groove. The apparatus also includes a restricting portion to hold the linear member between the restricting portion and the groove such that the supporting aperture leaf is controlled to be unmovable in the width direction and movable in the driving direction.

**Claims**

1. An apparatus for limiting an irradiation field, comprising:

a plurality of aperture leaves arranged in a width direction thereof, the aperture leaves being controlled so as to be unmovable in the width direction and movable in a driving direction substantially perpendicular to the width direction, the apparatus causing the aperture leaves to block radiations emitted by a radiation source such that the field to be irradiated is restricted to a desired area,

wherein at least one of the aperture leaves is a supporting aperture leaf having a groove at a periphery thereof, and the apparatus includes:

a linear member, one end of which is secured to the periphery of the supporting aperture leaf, the linear member engaging with the groove; and
a restricting portion configured to hold the linear member between the restricting portion and the groove such that the supporting aperture leaf is controlled so as to be unmovable in the width direction and movable in the driving direction.

**2.** The apparatus according to claim 1,
wherein the restricting portion comprises a roller provided with a groove on an external circumference thereof and supported rotatably in the driving direction of the supporting aperture leaf.

**3.** The apparatus according to claim 2, further comprising:

a connection unit connecting an adjacent pair of the aperture leaves such that the adjacent pair is configured to be unmovable in the width direction and movable in the driving direction,
wherein the aperture leaves include supporting aperture leaves and at least one intermediate aperture leaf that is placed between two of the supporting aperture leaves controlled by adjacent restricting portions, and
wherein the adjacent restricting portions, control the intermediate aperture leaf to be unmovable in the width direction and movable in the driving direction through the two of the supporting aperture leaves and the connection unit.

**4.** The apparatus according to claim 3, wherein the connection unit comprises:

a rolling portion disposed between the adjacent pair of the aperture leaves; and
a housing portion provided for at least one of the adjacent pair of the aperture leaves to accommodate the rolling portion.

**5.** The apparatus according to claim 1, wherein the linear member comprises:

a first forming portion; and

a second forming portion disposed adjacent to the first forming portion,

the linear member of the first forming portion being configured in a freely supported state such that the linear member lies more apart from the periphery of the supporting aperture leaf as a position of the linear member approaches another end differing from the secured one end of the linear member; and

the linear member of the second forming portion being configured in a freely supported state such that the linear member lies closer to the periphery of the supporting aperture leaf as a position of the linear member further approaches the other end from an end of the first forming portion.

**6.** The apparatus according to claim 1, further comprising a plurality of linear member driving devices that are disposed at different positions from one another when viewed in the width direction of the aperture leaves, each of the linear member driving devices including stacked unit drivers each driving another end differing from one secured end of a linear member in a direction of an axial line of the linear member.

**7.** The apparatus according to claim 6, wherein each of the linear member driving devices drives a group of linear members arranged at intervals of a predetermined number of linear members arranged in the width direction of the aperture leaves.

**8.** The apparatus according to claim 6, wherein
each of the unit drivers includes an actuator to rotationally drive a linear member, and
a central axis of a drive shaft of the actuator of one of the unit drivers and a central axis of a drive shaft of an actuator of at least another one of the unit drivers are configured to be substantially coaxial.

**9.** The apparatus according to claim 8, further comprising a drive force transmission unit configured to transmit a drive force of the actuator to the linear member, the drive force transmission unit comprising:

one of a drive wire and a drive belt to which the linear member is connected; and
a plurality of pulleys onto which the one of the drive wire and the drive belt is wound,
wherein the actuator is configured to rotationally drive one of the pulleys.

**10.** The apparatus according to claim 9, wherein each of the unit drivers comprises a tension adjustment unit configured to press the one of the drive wire and the drive belt so as to adjust a tension applied thereto.

**11.** The apparatus according to claim 9, further comprising:

> a stopper configured to restrict one of the aperture leaves from moving beyond a predetermined amount;
> a drive force detection unit configured to detect a drive force transmitted by the drive force transmission unit while the one of the aperture leaves is restricted by the stopper;
> a slippage detection unit configured to detect whether the one of the drive wire and the drive belt slips from the pulleys due to the transmitted drive force while the one of the aperture leaves is restricted by the stopper; and
> an output unit configured to output results of detection performed by the drive force detection unit and the slippage detection unit.

**12.** The apparatus according to claim 9, wherein the one of the drive wire and the drive belt is configured to slip at a surface thereof in contact with the pulleys due to the linear member receiving a predetermined tension.

**13.** The apparatus according to claim 6, further comprising:

> at least two position sensors that differ from each other in detection of a driven position of the linear member; and
> a controller configured to perform at least one of reducing a driving speed, reversing a driving direction and stopping a drive for the linear member when the controller compares signals sent from the at least two position sensors and detects a predetermined amount of difference therebetween.

**14.** The apparatus according to claim 13, wherein the at least two position sensors comprise:

> an absolute position sensor configured to detect an absolute position of the linear member; and
> a relative position sensor configured to detect an amount of the linear member driven with respect to a predetermined position.

**15.** A method for adjusting an apparatus for limiting an irradiation field, the method comprising the steps of:

> (a) restricting an aperture leaf by a stopper;
> (b) transmitting a drive force to the aperture leaf while the aperture leaf is restricted in step (a);
> (c) detecting the drive force transmitted to the aperture leaf in step (b) by a drive force detection unit;
> (d) determining whether the drive force detected in step (c) is out of a predetermined range by checking a signal sent to an output unit;
(e) detecting by a slippage detection unit whether the drive force transmitted in step (b) causes one of a drive wire and a drive belt to slip from a pulley;
(f) determining whether the one of the drive wire and the drive belt slips from the pulley detected in step (e) based on the signal sent to the output unit;
(g) adjusting a tension applied to the one of the drive wire and the drive belt,
wherein step (g) comprises the steps of:

> reducing the tension, when in step (d) the drive force is determined equal to or greater than an upper limit of the predetermined range and in step (f) no occurrence of slippage is determined; and
> increasing the tension, when in step (d) the drive force is determined equal to or smaller than a lower limit of the predetermined range and in step (f) an occurrence of slippage is determined.

**16.** The method according to claim 15, wherein in step (c), the drive force is detected by causing the drive force detection unit to be mechanically connected with the aperture leaf.

**17.** A linear member driving device for driving a plurality of linear members in directions of axial lines thereof, comprising:

> a plurality of stacked unit drivers, each being configured to drive each of the linear members, wherein each of the unit drivers includes an actuator to rotationally drive each of the linear members, and
> wherein a central axis of a drive shaft of the actuator and a central axis of a drive shaft of an actuator of at least another one of the unit drivers are substantially coaxially arranged.

**18.** The linear member driving device according to claim 17, further comprising a drive force transmission unit configured to transmit a drive force to one of the linear members,
wherein the drive force transmission unit comprises:

> one of a drive wire and a drive belt to which the one of the linear members is connected; and
> a plurality of pulleys onto which one of the drive wire and the drive belt is wound,
> wherein the actuator drives one of the pulleys to rotate.

**19.** The linear member driving device according to claim 18, further comprising a tension adjustment unit configured to press the one of the drive wire and the drive belt so as to adjust tension applied thereto.

20. The linear member driving device according to claim 18, wherein the one of the drive wire and the drive belt is configured to slip at a surface thereof in contact with the pulleys due to the one of the linear members receiving predetermined tension.

21. The linear member driving device according to claim 17, further comprising:

at least two position sensors differing from each other in detection of a driven position of the one of the linear members; and
a controller configured to compare signals sent from the at lest two position sensors and stop driving the one of the linear members when a predetermined difference exists between the signals.

22. The linear member driving device according to claim 21,
wherein the at least two sensors comprise:

an absolute position sensor configured to detect an absolute position of the one of the linear members; and
a relative position sensor configured to detect an amount of the one of the linear members driven with respect to a predetermined position.

FIG. 1

FIG. 2

# FIG. 3

FIG. 4

FIG. 5A

FIG. 5B

# FIG. 6

FIG. 7

CONTROL
UNIT

FIG. 8

FIG. 9

$$V_{out} = V_{in} \times \frac{x}{L}$$

(c-2)

(c-1)

(b)

(a)

EP 2 340 871 A2

# FIG. 10A

91  92 ~|MONITOR| 90  93

|STORAGE UNIT|  |CONTROL UNIT|  |INPUT UNIT|

41 ~ 46 ~|MOTOR|
41a ~ 46a ~|ENCODER|

SCREW 75

4A

LOAD CELL 94

94a

LINEAR MEMBER
31A ~ 36A

UNIT DRIVER
51 ~ 56

APERTURE BLADE
21A ~ 26A

# FIG. 10B

MEASURED DRIVE FORCE F1

F1-H

F1-L

REQUIRED RANGE
Range1

0

DRIVE VOLTAGE V1

# FIG. 11

METHOD 1 FOR ADJUSTING SLIP TORQUE

```
        ( START )
            │
            ▼
S10   ┌──────────────────────────────┐
      │ CAUSE APERTURE BLADE TO ABUT │
      │ WITH LOAD CELL (PLACEMENT STEP)│
      └──────────────────────────────┘
```

S20 — INCREASE DRIVE FORCE
(DRIVE FORCE TRANSMISSION STEP)
START MEASUREMENT OF DRIVEFORCE
(DRIVE FORCE DETECTION STEP)
START OUTPUT OF ENCODER
(SLIP DETECTION STEP)

S30 — DRIVE FORCE IS GREATER THAN OR EQUALTO UPPER LIMIT F1-H? (DRIVE FORCE DETERMINATION STEP)

YES → S31 — STOP DRIVING ADJUST TO REDUCE TENSION (TENSION REDUCTION STEP)

NO

S40 — SLIP OCCURRED? (SLIP DETERMINATION STEP)

NO

YES

S50 — DRIVE FORCE IS LESS THAN OR EQUAL TO LOWER LIMIT F1-L? (DRIVE FORCE DETERMINATION STEP)

YES → S51 — STOP DRIVING ADJUST TO INCREASE TENSION (TENSION INCREASE STEP)

NO

S60 — INPUT DRIVE VOLTAGE INDICATED DURING SLIP

S70 — ( END )

30

## FIG. 12A

91    92    MONITOR    90    93

STORAGE UNIT    CONTROL UNIT    INPUT UNIT

41 ~ 46    MOTOR

41a ~ 46a    ENCODER

SCREW 75

4A

STOPPER 95

LINEAR MEMBER    UNIT DRIVER
31A ~ 36A    51 ~ 56

APERTURE BLADE
21A ~ 26A

## FIG. 12B

CALCULATED DRIVE FORCE F2

REQUIRED RANGE Range2

F2-H

F2-L

0    V2-L  V2-H    DRIVE VOLTAGE V2

# FIG. 13

METHOD 2 FOR ADJUSTING SLIP TORQUE

START

S110 — CAUSE APERTURE BLADE TO ABUT WITH LOAD CELL (PLACEMENT STEP)

S120 — INCREASE DRIVE FORCE
(DRIVE FORCE TRANSMISSION STEP)
START MEASUREMENT OF DRIVEFORCE
(DRIVE FORCE DETECTION STEP)
START OUTPUT OF ENCODER
(SLIP DETECTION STEP)

S130 — DRIVE VOLTAGE IS GREATER THAN OR EQUAL TO UPPER LIMIT V2-H?
(DRIVE FORCE DETERMINATION STEP)

YES

S131 — STOP DRIVING
ADJUST TO REDUCE TENSION
(TENSION REDUCTION STEP)

NO

S140 — SLIP OCCURRED?
(SLIP DETERMINATION STEP)

NO

YES

S150 — DRIVE VOLTAGE IS LESS THAN OR EQUAL TO LOWER LIMIT V2-L?
(DRIVE FORCE DETERMINATION STEP)

YES

S151 — STOP DRIVING
ADJUST TO INCREASE TENSION
(TENSION INCREASE STEP)

NO

S160 — INPUT DRIVE VOLTAGE INDICATED DURING SLIP

S170 — END

# FIG. 14

# FIG. 15

FIG. 16

# FIG. 17

FIG. 18

EP 2 340 871 A2

FIG. 19

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009298045 A

- JP 10282 A